# EUROPEAN PATENT SPECIFICATION

(11) **EP 1 545 537 B1**
(45) Date of publication and mention of the grant of the patent: **04.04.2007**
(21) Application number: 03791540.2
(22) Date of filing: 01.09.2003
(51) Int. Cl.: A61K 31/505, A61K 31/439, A61K 31/40, A61K 31/351, A61K 31/22, A61K 31/404, A61K 31/4418, A61K 31/366, A61P 25/28

(54) **ALPHA-7 NICOTINIC RECEPTOR AGONISTS AND STATINS IN COMBINATION**
ALPHA-7-NIKOTINSÄURE-REZEPTORAGONISTEN UND STATINE IN KOMBINATION
AGONISTES DU RECEPTEUR NICOTINIQUE ALPHA-7 ET STATINES COMBINES

(30) Priority: 02.09.2002 SE 0202598
(43) Date of publication of application: 29.06.2005
(73) Proprietor: AstraZeneca AB, 151 85 Södertälje (SE)
(72) Inventor: KEITH, Richard, Wilmington, DE 19850-5437 (US)
(86) International application number: PCT/SE2003/001352
(87) International publication number: WO 2004/019947

(56) References cited:
- WO-A1-00/42044
- WO-A1-01/32620
- WO-A1-96/06098
- US-A- 6 110 914
- US-A1- 2002 042 429
- JICK H. ET AL.: 'Statins and the risk of dementia' LANCET vol. 356, 11 November 2000, pages 1627 - 1631, XP004264153
- BUXBAUM JOSEPH D. ET AL.: 'Pharmacological concentrations of the HMG-COA reductase inhibitor lovastatin decrease the formation of the Alzheimer beta-amyloid peptide in vitro and in patients' FRONTIERS IN BIOSCIENCE vol. 7, 01 April 2002, pages 50 - 59, XP002974322

## Description

### Field of Invention:

This invention is concerned with the treatment of neurological degenerative diseases and particularly with the treatment of Alzheimer's disease.

### Background

The etiology of Alzheimer's disease is complex and not entirely understood. Current hypotheses point to the overproduction of the amyloid peptide Aβ as a causative factor in the cognitive deficits and neurodegeneration associated with Alzheimer's disease (Selkoe, 2001; Walsh *et. al.,* 2002). In addition, epidemiological studies have shown that hypercholesterolemia is a risk factor for Alzheimer's disease (Jarvik *et. al.,* 1995; Notkola *et. al.,* 1998). Further, it has recently been shown that the administration of statins is associated with a decreased risk of Alzheimer's disease (Jick *et. al*., 2000; Wolozin *et. al.,* 2000). Still further, another recent study has shown that the statin lovastatin reduced Aβ plasma levels in human subjects that had elevated plasma levels of low-density lipoprotein cholesterol (Buxbaum *et. al.,* 2002).

Alpha-7 nicotinic receptors (α7-nAChR) are ligand-gated ion channels that allow for the entry into cells of calcium and other monovalent cations (Dani, 2001). α7-nAChR have been shown to play an important role in regulating neurotransmitter release, hippocampal synaptic function, neuroprotection against a variety of insults, and cognition (Dani, 2001; Dahas-Bailador *et. al.,* 2000; Rezvani and Levin, 2001).

Recent studies imply an interaction between Aβ and α7-nAChR that may contribute to the pathophysiology of Alzheimer's disease. Aβ has been shown to potently inhibit α7-nAChR (Liu *et. al.,* 2001). It has been proposed that this inhibitory effect of Aβ on α7-nAChR function may contribute to cognitive deficits in Alzheimer's disease. Neurodegeneration induced by the activation of NMDA glutamatergic receptors is also enhanced in the presence of Aβ (Kihara *et. al.,* 2001). This Aβ induced neurodegeneration is inhibited by activation of α7-nAChR.

### Background References:

Buxbaum JD, Cullen EI and Friedhoff LT: Pharmacological concentrations of the HMG-CoA reductase inhibitor lovastatin decrease the formation of the Alzheimer beta-amyloid peptide in vitro and in patients. *Frontiers in Bioscience* 7:a50-a59, 2002.

Dajas-Bailador FA, Lima PA and Wonnacott S: The α7 nicotinic receptor subtype mediates nicotine protection against NMDA excitotoxicity in primary hippocampal cultures through a Ca²⁺ dependent mechanism. *Neuropharmacology* 39:2799-2807, 2000.

Dani JA: Overview of nicotinic receptors and their roles in the central nervous system. *Biol Psychiatry* 49:166-174, 2001.

Kihara T, Shimohama S, Sawada H, Honda K, Nakamizo T, Shibasaki H, Toshiaki K, and Akaike A: a7 Nicotinic receptor transduces signals to phohphatidylinositol 3-kinase to block Aβ-amyloid-induced neurotoxicity. *J Biol Chem* 276:13541-13546, 1998.

Jick H, Zornberg GL, Jick SS, Seshadri S, and Drachman DA: Statins and the risk of dementia. *Lancet* 356:1627-1631, 2000.

Jarvik GP, Wijsman EM, Kukull WA, Schellenberg GD, Yu C and Larson EB: Interactions of apolipoprotein E genotype, total cholesterol, age, and sex in prediction of Alzheimer's *disease. Neurology* 45:1092-1096, 1995.

Liu Q, Kawai H and Berg DK: b-Amyloid peptide blocks the response of α7-containing nicotinic receptors on hippocampal neurons. *Proc Natl Acad Sci* 97:10197-10202, 2001.

Notkola IL, Sulkava R, Pekkanen J, Erkinjuntti T, Ehnholm C, Kivinen P, Tuomilehto J and Nissinen A: Serum total cholesterol, apolipoprotein E epsilon 4 allele, and Alzheimer's disease. *Neuroepidememiology* 17:14-20, 1998.

Rezvani AH and Levin ED: Cognitive effects of nicotine. *Biol Psychiatry* 49:258-267, 2001.

Selkoe, DJ: Alzheimer's disease: Genes, proteins, and therapy. *Physiological Reviews* 81:741-766, 2001.

Walsh DM, Klyubin I, Fadeeva JV, Cullen WK, and Selkoe, DJ: Naturally secreted oligomers of amyloid β protein potently inhibit hippocampal long-term potentiation in vivo. *Nature* 416:535-539, 2002.

Wolozin B, Kellman W, Ruosseau P, Celesia GG, and Siegel G: Decreased prevalence of Alzheimer's disease associated with 3-hydroxy-3-methyglutaryl coenzyme A reductase inhibitors. *Arch Neruol* 57:1439-1443, 2000.

### Description of the Invention:

We have discovered that statins and α7-nAChR agonists in combination have the potential to alter the pathophysiology of Alzheimer's disease and symptoms. The different mechanisms by which statins and α7-nAChR agonists operate - statins by reducing the formation of the neurotoxic substance Aβ and α7-nAChR agonists by blocking the cognitive impairing and neurotoxic effects of Aβ - imply that a statin and an α7-nAChR in combination will synergistically benefit patients suffering with neurological degenerative diseases and particularly patients suffering with Alzheimer's disease.

In one aspect the invention is a method for treating neurological degenerative diseases and particularly Alzheimer's disease comprising treatment with a combination comprising an α7-nAChR agonist and a statin.

A combination suitable for practicing the invention comprises a statin selected from atorvastatin, cerivastatin, fluvastatin, lovastatin, pravastatin sodium, simvastatin or rosuvastatin, or a pharmaceutically-acceptable salt thereof and an α7-nAChR agonist selected from spiro[1-azabicyclo[2.2.2]octane-3,5'-oxazolidine]-2'-one,
(+)-spiro[1-azabicyclo[2.2.2]octane-3,5'-oxazolidine]-2'-one,
(-)-spiro[1-azabicyclo[2.2.2]octane-3,5'-oxazolidine]-2'-one,
spiro[1-azabicyclo[2.2.1]heptan-3,5'-oxazolidin-2'-one],
3'-methyl spiro-[1-azabicyclo[2.2.2]octane-3,5'-oxazolidin-2'-one],
spiro[1-azabicyclo[2.2.2]octane-3,2'-(3'H)-furo[2,3-b]pyridine],
5'-bromospiro[1-azabicyclo[2.2.2]octane-3,2'-(3'H)furo[2,3-b]pyridine],
5'-phenylspiro[1-azabicyclo[2.2.2]octane-3,2'-(3'H)-furo(2,3-b]pyridine],
5'-nitrospiro[1-azabicyclo[2.2.2]octane-3,2'-(3'H)-furo[2,3-b]pyridine],
1'-chlorospiro[1-azabicyclo[2.2.2]octane-3,2'-(3'H)-furo[2,3-b]isoquinoline],
5'-(phenylcarboxamido)spiro[1-azabicyclo[2.2.2]octane-3,2'-(3'H)-furo[2,3-b]pyridine],
5'-(phenylaminocarbonylamino)spiro[1-azabicyclo[2.2.2]octane-3,2'-(3'H)-furo[2,3-b]pyridine],
5'-(phenylsulfonylamido)spiro[1-azabicyclo[2.2.2]octane-3,2'-(3'H)-furo[2,3-b]pyridine],
5'-aminospiro[1-azabicyclo[2.2.2]octane-3,2'-(3'H)-furo[2,3-b]pyridine),
5'-N-methylaminospiro[1-azabicyclo[2.2.2]octane-3,2'-(3'H)-furo[2,3-b]pyridine],
5'-N,N-dimethylaminospiro[1-azabicyclo[2.2.2]octane-3,2'-(3'H)-furo[2,3-b]pyridine],
5'-N,N-diethylaminospiro[1-azabicyclo[2.2.2]octane-3,2'-(3'H)-furo[2,3-b]pyridine],
5'-N-ethylaminospiro[1-azabicyclo[2.2.2]octane-3,2'-(3'H)-furo[2,3-b]pyridine],
5'-N-benzylaminospiro[1-azabicyclo[2.2.2]octane-3,2'-(3'H)-furo[2,3-b]pyridine],
5'-N-formamidospiro[1-azabicyclo[2.2.2]octane-3,2'-(3'H)-furo[2,3-b]pyridine],
5'-N-acetamidospiro[1-azabicyclo[2.2.2]octane-3,2'-(3'H)-furo[2,3-b]pyridine],
spiro[1-azabicyclo[2.2.2]octane-3,2'-(3'H)-furo[2,3-b]isoquinoline],
spiro[1-azabicyclo[2.2.2]octane-3,2'-(3'H)-furo[2,3-b]quinoline],
5'-ethenylspiro[1-azabicyclo[2.2.2]octane-3,2'-(3'H)-furo[2,3-b]pyridine],
5'-(E)-(phenylethenyl)spiro[1-azabicyclo[2.2.2]octane-3,2'-(3'H)-furo[2,3-b]pyridine],
5'-(4-morpholino)spiro[1-azabicyclo[2.2.2]octane-3,2'-(3'H)-furo[2,3-b]pyridine],
5'-(1-azetidinyl)spiro[1-azabicyclo[2.2.2]octane-3,2'-(3'H)-furo[2,3-b]pyridine],
5'-(E)-(2-(4-pyridyl)ethenyl)spiro[1-azabicyclo[2.2.2]octane-3,2'-(3'H)-furo[2,3-b]pyridine],
5'-(E)-(2-(2-pyridyl)ethenyl)spiro[1-azabicyclo[2.2.2]octane-3,2'-(3'H)-furo[2,3-b]pyridine],
5'-(2-trimethylsilylethynyl)spiro[1-azabicyclo[2.2.2]octane-3,2'-(3'H)-furo[2,3-b]pyridine],
5'-ethynylspiro[1-azabicyclo[2.2.2]octane-3,2'-(3'H)-furo[2,3-b]pyridine],
5'-(2-furyl)spiro[1-azabicyclo[2.2.2]octane-3,2'-(3'H)-furo[2,3-b]pyridine],
5'-(3-pyridyl)spiro[1-azabicyclo[2.2.2]octane-3,2'-(3'H)-furo[2,3-b]pyridine],
5'-methylspiro[1-azabicyclo[2.2.2]octane-3,2'-(3'H)-furo[2,3-b]pyridine],
spiro[1-azabicyclo[2.2.2]octane-3,2'-(3'H)-furo[2,3-b]pyridine-5'carbonitrile],
spiro[1-azabicyclo[2.2.2]octane-3,2'-(3'H)-furo[2,3-b]pyridine-5'carboxamide],
5'-N'-(3-chlorophenyl)aminocarbonylminospiro[1-azabicyclo[2.2.2]octane-3,2'-(3'H)-furo[2,3-b]pyridine],
5'-N'-(2-nitrophenyl)aminocarbonylaminospiro[1-azabicyclo[2.2.2]octane-3,2'-(3'H)-furo[2,3-b]pyridine],
4'-chlorospiro[1-azabicyclo[2.2.2]octane-3,2'-(3'H)-furo[2,3-b]pyridine],
4'-methoxyspiro[1-azabicyclo[2.2.2]octane-3,2'-(3'H)-furo[2,3-b]pyridine],
4'-phenylthiospiro[1-azabicyclo[2.2.2]octane-3,2'-(3'H)-furo[2,3-b]pyridine],
4'-(N-2-aminoethyl)aminospiro[1-azabicyclo[2.2.2]octane-3,2'-(3'H)-furo[2,3-b]pyridine],
4'-phenylaminospiro[1-azabicyclo[2.2.2]octane-3,2'-(3'H)-furo[2,3-b]pyridine],
4'-methylaminospiro[1-azabicyclo[2.2.2]octane-3,2'-(3'H)-furo[2,3-b]pyridine],
4'-(4-N-methylpiperazin-1-yl)spiro[1-azabicyclo[2.2.2]octane-3,2'-(3'H)-furo[2,3-b]pyridine],
4'-chloro-spiro[1-azabicyclo[2.2.2]octane-3,2'-(3'H)-furo[3,2-c]pyridine],
spiro[1-azabicyclo[2.2.2]octane-3,2'-(3'H)-furo[3,2-c]pyridine],
spiro[1-azabicyclo[2.2.2]octane-3,2'(3'H)-furo[2,3-b]pyridine-7'-oxide],
spiro[1-azabicyclo[2.2.2]octane-3,2'(3'H)-furo[2,3-b]pyridine-6'-carbonitrile],
6'-chlorospiro[1-azabicyclo[2.2.2]octane-3,2'(3'H)-furo[2,3-b]pyridine],
6'-fluorospiro[1-azabicyclo[2.2.2]octane-3,2'(3'H)-furo[2,3-b]pyridine],
*N*-(1-azabicyclo[2.2.2]oct-3-yl)(5-phenylfuran-2-carboxamide),
*N*-(1-azabicyclo[2.2.2]oct-3-yl)(5-(3-fluotophenyl)furan-2-carboxamide),
*N*-(1-azabicyclo[2.2.2]oct-3-yl)(3-(3-thienyl)benzamide),
*N*-(1-azabicyclo[2.2.2]oct-3-yl)(3-phenylbenzamide),
*N*-(1-azabicyclo[2.2.2]oct-3-yl)(3-(3-pyridyl)benzamide),
*N*-(1-azabicyclo[2.2.2]oct-3-yl)(5-phenylthiophene-2-carboxamide),
*N*-(1-azabicyclo[2.2.2]oct-3-yl)(3-(3-methoxyphenyl)benzamide),
*N*-(1-azabicyclo[2.2.2]oct-3-yl)(3-(2-methoxyphenyl)benzamide),
*N*-(1-azabicyclo[2.2.2]oct-3-yl)(3-(3-(*N*-acetylamino)phenyl)benzamide),
*N*-(1-azabicyclo[2.2.2]oct-3-yl)(3-(3-fluorophenyl)benzamide),
*N*-(1-azabicyclo[2.2.2]oct-3-yl)(3-(3-methylphenyl)benzamide),
*N*-(1-azabicyclo[2.2.2]oct-3-yl)(3-(2-thienyl)benzamide),
*N*-(1-azabicyclo[2.2.2]oct-3-yl)(3-(3,5-dichlorophenyl)benzamide),
*N*-(1-azabicyclo[2.2.2]oct-3-yl)(3-(2-naphthyl)benzamide),
*N*-(1-azabicyclo[2.2.2]oct-3-yl)(3-(4-fluorophenyl)benzamide),
*N*-(1-azabicyclo[2.2.2]oct-3-yl)(5-(3-pyridyl)furan-2-carboxamide),
*N*-(1-azabicyclo[2.2.2]oct-3-yl)(5-(3-thienyl)furan-2-carboxamide),
*N*-(1-azabicyclo[2.2.2]oct-3-yl)(5-(2-benzo[b]furanyl)furan-2-carboxamide),
*N*-(1-azabicyclo[2.2.2]oct-3-yl)(5-(4-pyridyl)furan-2-carboxamide),
*N*-(1-azabicyclo[2.2.2]oct-3-yl)(5-(2-thienyl)furan-2-carboxamide),
*N*-(1-azabicyclo[2.2.2]oct-3-yl)(5-(3-methoxyphenyl)furan-2-carboxamide),
*N-*(1-azabicyclo[2.2.2]oct-3-yl)(5-(2-methoxyphenyl)furan-2-carboxamide),
*N*-(1-azabicyclo[2.2.2]oct-3-yl)(5-(4-fluorophenyl)furan-2-carboxamide),
*N*-(1-azabicyclo[2.2.2]oct-3-yl)(5-(2-naphthyl)furan-2-carboxamide),
*N*-(1-azabicyclo[2.2.2]oct-3-yl)(5-(3-methylphenyl)furan-2-carboxamide),
*N*-(1-azabicyclo[2.2.2]oct-3-yl)(5-(3-furyl)furan-2-carboxamide),
*N*-(1-azabicyclo[2.2.2]oct-3-yl)(5-(2-furyl)furan-2-carboxamide),
*N*-(1-azabicyclo[2.2.2]oct-3-yl)(5-(2-pyridyl)furan-2-carboxamide),
*N*-(1-azabicyclo[2.2.2]oct-3-yl)(5-(4-pyridyl)thiophene-2-carboxamide);
*N*-(1-azabicyclo[2.2.2]oct-3-yl)(5-(3-pyridyl)thiophene-2-carboxamide),
*N*-(1-azabicyclo[2.2.2]oct-3-yl)(5-(2-pyridyl)thiophene-2-carboxamide),
*N*-(1-azabicyclo[2.2.2]oct-3-yl)(4-(2-pyridyl)thiophene-2-carboxamide),
*N*-(1-azabicyclo[2.2.2]oct-3-yl)(4-(4-pyridyl)thiophene-2-carboxamide),
*N*-(1-azabicyclo[2.2.2]oct-3-yl)(4-(3-pyridyl)thiophene-2-carboxamide),
*N*-(1-azabicyclo[2.2.2]oct-3-yl)(5-(3-(*N*-acetylamino)phenyl)furan-2-carboxamide),
*N*-(1-azabicyclo[2.2.2]oct-3-yl)(5-(3-nitrophenyl)furan-2-carboxamide),
*N*-(1-azabicyclo[2.2.2]oct-3-yl)(5-(3-trifluoromethylphenyl)furan-2-carboxamide),
*N*-(1-azabicyclo[2.2.2]oct-3-yl)(5-(3-chlorophenyl)furan-2-carboxamide),
*N*-(1-azabicyclo[2.2.2]oct-3-yl)(5-(3-(*N*-acetylamino)phenyl)thiophene-2-carboxamide),
*N*-(1-azabicyclo[2.2.2]oct-3-yl)(5-(3-fluorophenyl)thiophene-2-carboxamide),
*N*-(1-azabicyclo[2.2.2]oct-3-yl)(5-(3-methoxyphenyl)thiophene-2-carboxamide),
*N*-(1-azabicyclo[2.2.2]oct-3-yl )(5-(3-ethoxyphenyl)thiophene-2-carboxamide),
*N*-(1-azabicyclo[2.2.2]oct-3-yl)(5-(3,5-dimethylisoxazol-4-yl)furan-2-carboxamide),
*N*-(1-azabicyclo[2.2.2]oct-3-yl)(5-(3,5-dimethylisoxazol-4-yl)thiophene-2-carboxamide),
*N*-(1-azabicyclo[2.2.2]oct-3-yl)(5-(3-aminophenyl)thiophene-2-carboxamide),
*N*-(1-azabicyclo[2.2.2]oct-3-yl)(5-(3-pyridyl)thiophene-3-carboxamide),
*N*-(1-azabicyclo[2.2.2]oct-3-yl)(5-(4-chlorophenyl)furan-2-carboxamide),
*N*-(1-azabicyclo[2.2.2]oct-3-yl)(5-(3-pyridyl)thiazole-3-carboxamide),
*N*-(1-azabicyclo[2.2.2]oct-3-yl)(5-(4-pyridyl)thiazole-3-carboxamide),
N-(1-azabicyclo[2.2.2]oct-3-yl)(5-(3-(*N,N*-dimethylamino)phenyl)thiophene-2-carboxamide),
*N*-(1-azabicyclo[2.2.2]oct-3-yl)(5-(8-quinolinyl)thiophene-2-carboxamide),
*N*-(1-azabicyclo[2.2.2]oct-3-yl)(5-phenylthiophene-3-carboxamide),
*N-*(1-azabicyclo[2.2.2]oct-3-yl)(4-phenylthiophene-2-carboxamide),
*N*-(1-azabicyclo[2.2.2]oct-3-yl)(5-(3-cyanophenyl)thiophene-2-carboxamide),
*N*-(1-azabicyclo[2.2.2]oct-3-yl)(5-(3-(N-methylamino)phenyl)thiophene-2-carboxamide),
*N*-(1-azabicyclo[2.2.2]oct-3-yl)(5-(3-hydroxyphenyl)thiophene-2-carboxamide),
*N*-(1-azabicyclo[2.2.2]oct-3-yl)(5-(3-pyridylamino)thiophene-2-carboxamide),
*N*-(1-azabicydo[2.2.2]oct-3-yl)(5-(3-chlorophenyl)thiophene-2-carboxamide),
*N*-(1-aza-bicyclo[2.2.2]oct-3-yl)(5-(3-(4-morpholinyl)phenyl)thiophene-2-carboxamide),
*N*-(1-azabicyclo[2.2.2]oct-3-yl)(5-(3-(aminomethyl)phenyl)thiophene-2-carboxamide),
*N*-(1-azabicyclo[2.2.2]oct-3-yl)(5-phenoxythiophene-2-carboxamide),
*N*-(1-azabicyclo[2.2.2]oct-3-yl)(5-(3-aminophenyl)furan-2-carboxamide),
*N*-(1-azabicyclo[2.2.2]oct-3-yl)(5-(3-(*N*,*N*-dimethylamino)phenyl)furan-2-carboxamide),
*N*-(1-azabicyclo[2.2.2]oct-3-yl)(5-(3-formylphenyl)thiophene-2-carboxamide); *N-*(1-azabicyclo[2.2.2]oct-3-yl)(5-(3-(hydroxymethyl)phenyl)thiophene-2-carboxamide),
(*R*)-*N*-(1-azabicyclo[2.2.2]oct-3-yl)(5-phenylfuran-2-carboxamide),
(*R*)-*N*-(1-azabicyclo[2.2.2]oct-3-yl)(5-(3-fluorophenyl)furan-2-carboxamide),
(*R*)*-N-*(1-azabicyclo[2.2.2]oct-3-yl)(3-(3-thienyl)benzamide),
(*R*)-*N*-(1-azabicyclo[2.2.2]oct-3-yl)(3-phenylbenzamide),
(*R*)-*N*-(1-azabicyclo[2.2.2]oct-3-yl)(3-(3-pyridyl)benzamide),
(*R*)-*N*-(1-azabicyclo[2.2.2]oct-3-yl)(5-phenylthiophene-2-carboxamide),
(*R*)-*N*-(1-azabicyclo[2.2.2]oct-3-yl)(3-(3-methoxyphenyl)benzamide),
(*R*)-*N*-(1-azabicyclo[2.2.2]oct-3-yl)(3-(2-methoxyphenyl)benzamide),
(*R*)-*N*-(1-azabicyclo[2.2.2]oct-3-yl)(3-(3-(*N*-acetylamino)phenyl)benzamide);
(*R*)-*N*-(1-azabicyclo[2.2.2]oct-3-yl)(3-(3-fluorophenyl)benzamide),
(*R*)-*N*-(1-azabicyclo[2.2.2]oct-3-yl)(3-(3-methylphenyl)benzamide),
(*R*)-*N*-(1-azabicyclo[2.2.2]oct-3-yl)(3-(2-thienyl)benzamide),
(*R*)-*N*-(1-azabicyclo[2.2.2]oct-3-yl)(3-(3,5-dichlorophenyl)benzamide),
(*R*)-*N*-(1-azabicyclo[2.2.2]oct-3-yl)(3-(2-naphthyl)benzamide),
(*R*)-*N*-(1-azabicyclo[2.2.2]oct-3-yl)(3-(4-fluorophenyl)benzamide),
(*R*)-*N*-(1-azabicyclo[2.2.2]oct-3-yl)(5-(3-pyridyl)furan-2-carboxamide),
(*R*)-*N*-(1-azabicyclo[2.2.2]oct-3-yl)(5-(3-thienyl)furan-2-carboxamide),
(*R*)-*N*-(1-azabicyclo[2.2.2]oct-3-yl)(5-(2-benzo[b]furanyl)furan-2-carboxamide),
(*R*)-*N*-(1-azabicyclo[2.2.2]oct-3-yl)(5-(4-pyridyl)furan-2-carboxamide),
(*R*)-*N*-(1-azabicyclo[2.2.2]oct-3-yl)(5-(2-thienyl)furan-2-carboxamide),
(*R*)-*N*-(1-azabicyclo[2.2.2]oct-3-yl)(5-(3-methoxyphenyl)furan-2-carboxamide),
(*R*)-*N*-(1-azabicyclo[2.2.2]oct-3-yl)(5-(2-methoxyphenyl)furan-2-carboxamide),
(*R*)-*N*-(1-azabicyclo[2.2.2]oct-3-yl)(5-(4-fluorophenyl)furan-2-carboxamide),
(*R*)-*N*-(1-azabicyclo[2.2.2]oct-3-yl)(5-(2-naphthyl)furan-2-carboxamide),
(*R*)-*N*-(1-azabicyclo[2.2.2]oct-3-yl)(5-(3-methylphenyl)furan-2-carboxamide),
(*R*)-*N*-(1-azabicyclo[2.2.2]oct-3-yl)(5-(3-furyl)furan-2-carboxamide),
(*R*)-*N*-(1-azabicyclo[2.2.2]oct-3-yl)(5-(2-furyl)furan-2-carboxamide),
(*R*)-*N*-(1-azabicyclo[2.2.2]oct-3-yl)(5-(2-pyridyl)furan-2-carboxamide),
*(R)-N*-(1-azabicyclo[2 .2.2]oct-3-yl)(5-(4-pyridyl)thiophene-2-carboxamide),
*(R)-N-*(1-azabicyclo[2.2.2]oct-3-yl)(5-(3-pyridyl)thiophene-2-carboxamide),
*(R)-N-*(1-azabicyclo[2.2.2]oct-3-yl)(5-(2-pyridyl)thiophene-2-carboxamide),
*(R)-N-*(1-azabicyclo[2.2.2]oct-3-yl)(4-(2-pyridyl)thiophene-2-carboxamide),
*(R)-N-*(1-azabicyclo[2.2.2]oct-3-yl)(4-(4-pyridyl)thiophene-2-carboxamide),
*(R)*-*N*-(1-azabicyclo[2.2.2]oct-3-yl)(4-(3-pyridyl)thiophene-2-carboxamide),
*(R)-N-*(1-azabicyclo[2.2.2]oct-3-yl)(5-(3-(*N*-acetylamino)phenyl)furan-2-carboxamide),
*(R)-N*-(1-azabicyclo[2.2.2]oct-3-yl)(5-(3-nitrophenyl)furan-2-carboxamide),
*(R)-N*-(1-azabicydo[2.2.2]oct-3-yl)(5-(3-trifluoromethylphenyl)furan-2-carboxamide),
(*R*)-*N*-(1-azabicyclo[2.2.2]oct-3-yl)(5-(3-chlorophenyl)furan-2-carboxamide),
(*R*)-*N*-(1-azabicyclo[2.2.2]oct-3-yl)(5-(3-(*N*-acetylamino)phenyl)thiophene-2-carboxamide),
(*R*)-*N*-(-azabicyclo[2.2.2]oct-3-yl)(5-(3-fluorophenyl)thiophene-2-carboxamide),
(*R*)-*N*-(-azabicyclo[2.2.2]oct-3-yl)(5-(3-methoxyphenyl)thiophene-2-carboxamide),
*(R)-N*-(1-azabicyclo[2.2.2]oct-3-yl)(5-(3-ethoxyphenyl)thiophene-2-carboxamide),
(*R*)-*N*-(1-azabicyclo[2.2.2]oct-3-yl)(5-(3,5-dimethylisoxazol-4-yl)furan-2-carboxamide),
(*R*)-*N*-(1-azabicyclo[2.2.2]oct-3-yl)(5-(3,5-dimethylisoxazol-4-yl)thiophene-2-carboxamide),
(*R*)-*N*-(1-azabicyclo[2.2.2]oct-3-yl)(5-(3-aminophenyl)thiophene-2-carboxamide),
(*R*)-*N*-(1-azabicyclo[2.2.2]oct-3-yl)(5-(3-pyridyl)thiophene-3-carboxamide),
(*R*)-*N*-(1-azabicyclo[2.2.2]oct-3-yl)[5-(4-chlorophenyl)furan-2-carboxamide),
(*R*)-*N*-(1-azabicyclo[2.2.2]oct-3-yl)(5-(3-pyridyl)thiazole-3-carboxamide),
(*R*)-*N*-(1-azabicyclo[2.2.2]oct-3-yl)(5-(4-pyridyl)thiazole-3-carboxamide),
(*R*)-*N*-(1-azabicyclo[2.2.2]oct-3-yl)(5-(3-(*N*,*N*-dimethylamino)phenyl)thiophene-2-carboxamide),
(*R*)-*N*-(1-azabicyclo[2.2.2]oct-3-yl)(5-(8-quinolinyl)thiophene-2-carboxamide),
(*S*)-*N*-(1-azabicyclo[2.2.2]oct-3-yl)(5-(3-pyridyl)thiophene-2-carboxamide);
(*S*)-*N*-(1-azabicyclo[2.2.2]oct-3-yl)(5-(4-pyridyl)thiophene-2-carboxamide),
(*S*)-*N*-(1-azabicyclo[2.2.2]oct-3-yl)(5-(2-pyridyl)thiophene-2-carboxamide),
(*S*)-*N*-(1-azabicyclo[2.2.2]oct-3-yl)(5-phenylthiophene-2-carboxamide),
*(R)-N*-(1-azabicyclo[2.2.2]oct-3-yl)(5-phenylthiophene-3-carboxamide),
*(R)-N*-(1-azabicyclo[2.2.2]oct-3-yl)(4-phenylthiophene-2-carboxamide),
*(R)-N-*(1-azabicyclo[2.2.2]oct-3-yl)(5-(3-cyanophenyl)thiophene-2-carboxamide),
*(R)-N*-(1-azabicyclo[2.2.2]oct-3-yl)(5-(3-(*N*-methylamino)phenyl)thiophene-2-carboxamide),
*(R)*-*N*-(1-aza-bicyclo[2.2.2]oct-3-yl)(5-(3-hydroxyphenyl)thiophene-2-carboxamide),
*(R)-N-*(1-azabicyclo[2.2.2]oct-3-yl)(5-(3-pyridylamino)thiophene-2-carboxamide),
*(R)-N*-(1-azabicyclo[2.2.2]oct-3-yl)(5-(3-chlorophenyl)thiophene-2-carboxamide),
*(R)-N*-(1-azabicyclo[2.2.2]oct-3-yl)(5-(3-(4-morpholinyl)phenyl)thiophene-2-carboxamide),
*(R)-N*-(1-azabicyclo[2.2.2]oct-3-yl)(5-(3-(aminomethyl)phenyl)thiophene-2-carboxamide),
*(R)-N*-(1-azabicyclo[2.2.2]oct-3-yl)(5-phenoxythiophene-2-carboxamide),
*(R)-N*-(1-azabicyclo[2.2.2]oct-3-yl)(5-(3-aminophenyl)furan-2-carboxamide),
*(R)-N*-(1-azabicyclo[2.2.2]oct-3-yl)(5-(3-(*N,N*-dimethylamino)phenyl)furan-2-carboxamide),
*(R)-N-*(1-azabicyclo[2.2.2]oct-3-yl)(5-(3-formylphenyl)thiophene-2-carboxamide), or
(*R*)-*N*-(1-azabicyclo[2.2.2]oct-3-yl)(5-(3-(hydroxymethyl)phenyl)thiophene-2-carboxamide), or a pharmaceutically-acceptable salt thereof.

In general, it is contemplated that any statin when used in combination with any alpha-7-nAChR agonist will be useful in practicing the present invention.

Alpha-7-nAChR agonists contemplated to be useful in the present invention are described in international publications W09606098, WO9730998, WO 9903859, W09956745, WO0042044; WO0129034, W00160821, W00132622, WO0136417, WO0132619, WO0132620, WO0136417, WO0244176, WO0220521, WO0216358, WO0216357, W00216356, WO0216355, WO0215662 and W00217358 and in publications EP1219622, EP1184383, EP1184384, EP1184385, JP200203084. Statins contemplated to be useful in the present inventions are atorvastatin calcium (Lipitor), cerivastatin sodium (Baycol), fluvastatin sodium (Lescol), lovastatin (Mevacor), pravastatin sodium (Pravachol), simvastatin (Zocor) and rosuvastatin (Crestor).

In another aspect the invention is a pharmaceutical composition comprising a combination of an α7-nAChR agonist and a statin as described herein together with a pharmaceutically-acceptable diluent or excipient.

In another aspect the present invention comprises the use of a therapeutically effective amount of a combination as defined in Claim 1 for the manufacture of a medicament for providing neuroprotection or analgesia in the treatment or prophylaxis of a condition or disorder involving reduced cholinergic function selected from Alzheimer's disease, cognitive or attention disorders, anxiety, depression, smoking cessation, schizophrenia, Tourette's syndrome, and Parkinson's disease.

In a particular aspect the use of the invention is a use for the treatment or prophylaxis of Alzheimer's disease.

A further aspect of the invention is the use of a combination of an α7-nAChR agonist and a statin as described herein in the preparation of a medicament for providing neuroprotection or analgesia in the treatment of a condition or disorder involving reduced cholinergic function selected from Alzheimer's disease, cognitive or attention disorders, anxiety, depression, smoking cessation, schizophrenia, Tourette's syndrome, and Parkinson's disease.

In a particular aspect the use of a combination of an α7-nAChR agonist and a statin as described herein is in the preparation of a medicament for the treatment or prophylaxis of Alzheimer's disease.

A particular combination for use in the present invention comprises rosuvastatin or a pharmaceutically-acceptable salt thereof and an α7-nAChR agonist selected from spiro[1-azabicyclo[2.2.2]octane-3,5'-oxazolidine]-2'-one, *N*-(1-azabicyclo[2.2.2]oct-3-yl)[*E*-3-(2-thienyl)propenamide], or (2'R)-5'-(3-furanyl)spiro[1-azabicyclo[2.2.2]octane-3,2'-(3'H)-furo[2,3-b]pyridine] or a pharmaceutically-acceptable salt thereof.

A particular pharmaceutical composition for use in the present invention comprises rosuvastatin or a pharmaceutically-acceptable salt thereof and an α7-nAChR agonist selected from spiro[1-azabicyclo[2.2.2]octane-3,5'-oxazolidine]-2'-one, N-(1-azabicyclo[2.2.2]oct-3-yl)[*E*-3-(2-thienyl)propenamide], or (2'R)-5'-(3-furanyl)spiro[1-azabicyclo[2.2.2]octane-3,2'-(3'H)-furo[2,3-b]pyridine] or a pharmaceutically-acceptable salt thereof together with a pharmaceutically acceptable diluent or carrier.

A particular method of the present invention is the provision of neuroprotection or analgesia for the treatment or prophylaxis of a condition or disorder involving reduced cholinergic function selected from Alzheimer's disease, cognitive or attention disorders, anxiety, depression, smoking cessation, schizophrenia, Tourette's syndrome, and Parkinson's disease which method comprises administering a therapeutically effective amount of a combination of rosuvastatin or a pharmaceutically-acceptable salt thereof and an α7-nAChR agonist selected from spiro[1-azabicyclo[2.2.2]octane-3,5'-oxazolidine]-2'-one, *N*-(1-azabicyclo[2.2.2]oct-3-yl)[*E*-3-(2-thienyl)propenamide], or (2'R)-5'-(3-furanyl)spiro[1-azabicyclo[2.2.2]octane-3,2'-(3'H)-furo[2,3-b]pyridine] or a pharmaceutically-acceptable salt thereof to a patient. In particular the method is useful for the treatment or prophylaxis Alzheimer's disease.

A particular embodiment of the invention is the use of a combination rosuvastatin or a pharmaceutically-acceptable salt thereof and an α7-nAChR agonist selected from spiro[1-azabicyclo[2.2.2]octane-3,5'-oxazolidine]-2'-one, *N*-(1-azabicyclo[2.2.2]oct-3-yl)[*E*-3-(2-thienyl)propenamide], or (2'R)-5'-(3-furanyl)spiro[1-azabicyclo[2.2.2]octane-3,2'-(3'H)-furo[2,3-b]pyridine] or a pharmaceutically-acceptable salt thereof in the preparation of a medicament providing neuroprotection or analgesia for the treatment of a condition or disorder involving reduced cholinergic function selected from Alzheimer's disease, cognitive or attention disorders, anxiety, depression, smoking cessation, schizophrenia, Tourette's syndrome, and Parkinson's disease. In particular the invention comprises the use of such a combination in the preparation of a medicament for the treatment of Alzheimer's disease.

Statins are compounds that inhibit HMG-CoA reductase, a rate-limiting enzyme in the biosynthetic pathway to cholesterol. Statins are conventionally used to reduce plasma levels of cholesterol in patients with cardiovascular disease but can also reduce Aβ serum levels in patients. Alpha-7-nAChR agonists beneficially activate α7-nACh receptors and are useful for treating cognitive deficits and in the treatment of a range of disorders involving reduced cholinergic function such as Alzheimer's disease, cognitive or attention disorders, anxiety, depression, smoking cessation, neuroprotection, schizophrenia, analgesia, Tourette's syndrome, and Parkinson's disease. Accordingly, the hypothetical basis of the present invention lies in the realization that statins, by reducing the formation of Aβ, may be particularly effective in combination with α7-nAChR agonists, which ameliorate cognitive deficits and inhibit neurodegeneration induced by Aβ, in the treatment of Alzheimer's disease. Therefore, the treatment of Alzheimer's disease with a combination of a statin and an α7-nAChR agonist will result in enhanced efficacy over either type of agent if administered alone.

### Experimental:

Assessment of the efficacy of a statin and an α7-nAChR agonist in combination in animal models is not straightforward. Existing experimental models of Alzhiemer's disease include transgenic mice, which over express Aβ, and animals with surgically generated fimbria-fornix lesions. These models and the uses to which they may be put are known, understood and appreciated by those of skill in the relevant art. Transgenic mice which over express Aβ exhibit some of the clinical manifestations of Alzheimer's disease, e.g., plaque deposition and, in some cases, cognitive deficits, but neurodegeneration is not observed. Animals with fimbria-fornix lesions have cognitive and learning deficits and have been used to assess potential approaches to treat neurodegeneration. No single experimental model exhibits the entire pathophysiological complex of Alzheimer's disease. However, to the extent that these models do mimic the pathophysiology of Alzheimer's disease they may be used to assess the effect of a statin and an α7-nAChR agonist in combination.

## Claims

1. A combination comprising:
a statin selected from atorvastatin, cerivastatin, fluvastatin, lovastatin, pravastatin, sodium, simvastatin or rosuvastatin, or a pharmaceutically-acceptable salt thereof and
an α7-nAChR agonist selected from spiro[1-azabicyclo[2.2.2]octane-3,5'-oxazolidine]-2'-one,
(+)-spiro[1-azabicyclo[2.2.2]octane-3,5'-oxazolidine]-2'-one,
(-)-spiro[1-azabicyclo[2.2.2]octane-3,5'-oxazolidine]-2'-one,
spiro[1-azabicyclo[2.2.1]heptan-3,5'-oxazolidin-2'-one],
3'-methyl spiro-[1-azabicyclo[2.2.2]octane-3,5'-oxazolidin-2'-one],
spiro[1-azabicyclo[2.2.2] octane-3,2'-(3'H)-furo[2,3-b]pyridine],
5'-bromospiro[1-azabicyclo[2.2.2]octane-3,2'-(3'H)-furo[2,3-b]pyridine],
5'-phenylspiro[1-azabicyclo[2.2.2]octane-3,2'-(3'H)-furo[2,3-b]pyridine],
5'-nitrospiro[1,-azabicyclo[2.2.2]octane-3,2'-(3'H)-furo[2,3-b]pyridine],
1'-chlorospiro[1-azabicyclo[2.2.2]octane-3,2'-(3'H)-furo[2,3-b]isoquinoline],
5'-(phenylcarboxamido)spiro[1-azabicyclo[2.2.2]octane-3,2'-(3'H)-furo[2,3-b]pyridine],
5'-(phenylaminocarbonylamino)spiro[1-azabicyclo[2.2.2]octane-3,2'-(3'H)-furo[2,3-b]pyridine],
5'-(phenylsulfonylamido)spiro[1-azabicyclo[2.2.2]octane-3,2'-(3'H)-furo[2,3-b]pyridine],
5'-aminospiro[1-azabicyclo[2.2.2]octane-3,2'-(3'H)-furo[2,3-b]pyridine],
5'-N-methylaminospiro[1-azabicyclo[2.2.2]octane-3,2'-(3'H)-furo[2,3-b]pyridine],
5'-N,N-dimethylaminospiro[1-azabicyclo[2.2.2]octane-3,2'-(3'H)-furo[2,3-b]pyridine],
5'-N,N-diethylaminospiro[1-axabicyclo[2.2.2]octane-3,2'-(3'H)-furo[2,3-b]pyridine],
5'-N-ethylaminospiro[1-azabicyclo[2.2.2]octane-3,2'-(3'H)-furo[2,3-b]pyridyne],
5'-N-benzylaminospiro[1-azabicyclo[2.2.2]octane-3,2'-(3'H)-furo[2,3-b]pyridine],
5'-N-formamidospiro[1-azabicyclo[2.2.2]octane-3,2'-(3'H)-furo[2,3-b]pyridine],
5'-N-acetamidospiro[1-azabicyclo[2.2.2]octane-3,2'-(3'H)-furo[2,3-b]ppidine],
spiro[1-azabicyclo[2.2.2]octane-3,2'-(3'H)-furo[2,3-b]isoquinoline],
spiro[1-azabicyclo[2.2.2]octane-3,2'-(3'H)-furo[2,3-b]quinoline],
5'-ethenylspiro[1-azabicyclo[2.2.2]octane-3,2'-(3'H)-furo[2,3-b]pyridine],
5'-(E)-(phenylethenyl)spiro[1-azabicyclo[2.2.2]octane-3,2'-(3'H)-furo[2,3-b]pyridine],
5'-(4-morpholino)spiro[1-azabicyclo[2.2.2]octane-3,2'-(3'H)-furo[2,3-b]pyridine],
5'-(1-azetidinyl)spiro[1-azabicyclo[2.2.2]octane-3,2'-(3'H)-furo[2,3-b]pyridine],
5'-(E)-(2-(4-pyridyl)ethenyl)spiro[1-azabicyclo[2.2.2]octane-3,2'-(3'H)-furo[2,3-b]pyridine],
5'-(E)-(2-(2-pyridyl)ethenyl)spiro[1-azabicyclo[2.2.2]octane-3,2'-(3'H)-furo[2,3-b]pyridine],
5'-(2-trimethylsilylethynyl)spiro[1-azabicyclo[2.2.2]octane-3,2'-(3'H)-furo[2,3-b]pyridine],
5'-ethynylspiro[1-azabicyclo[2.2.2]octane-3,2'-(3'H)-furo[2,3-b]pyridine],
5'-(2'-furyl)spiro[1-azabicyclo[2.2.2]octane-3,2'-(3'H)-furo[2,3-b]pyridine],
5'-(3-pyridyl)spiro[1-azabicyclo[2.2.2]octane-3,2'-(3'H)-furo[2,3-b]pyridine],
5'-methylspiro[1-azabicyclo[2.2.2]octane-3,2'-(3'H)-furo[2,3-b]pyridine],
spiro[1-azabicyclo[2.2.2]octane-3,2'-(3'H)-furo[2,3-b]pyridine-5'carbonitrile],
spiro[1-azabicyclo[2.2.2]octane-3,2'-(3'H)-furo[2,3-b]pyridine-5'carboxamide],
5'-N'-(3-chlorophenyl)aminocarbonylminospiro[1-azabicyclo[2.2.2]octane-3,2'-(3'H)-furo[2,3-b]pyridine],
5'-N'-(2-nitrophenyl)aminocarbonylaminospiro[1-azabicyclo[2.2.2] octane-3,2'-(3'H)-furo[2,3-b]pyridine],
4'-chlorospiro[1-azabicyclo[2.2.2]octane-3,2'-(3'H)-furo[2,3-b]pyridine],
4'-methoxyspiro[1-azabicyclo[2.2.2]octane-3,2'-(3'H)-furo[2,3-b]pyridine],
4'-phenylthiospiro[1-azabicyclo[2.2.2]octane-3,2'-(3'H)-fur[2,3-b]pyridine],
4'-N-2-aminoethyl)aminospiro[1-azabicyclo[2.2.2]octane-3,2'-(3'H)-furo[2,3-b]pyridine],
4'-phenylaminospiro[1-azabicyclo[2.2.2]octane-3,2'-(3'H)-furo[2,3-b]pyridine],
4'-methylaminospiro[1-azabicyclo[2.2.2]octane-3,2'-(3'H)-furo[2,3-b]pyridine],
4'-(4-N-methylpiperazin-1-yl)spiro[1-azabicyclo[2.2.2]octane-3,2'-(3'H)-furo[2,3-blpyridine],
4'-chloro-spiro[1-azabicyclo[2.2.2]octane-3,2'-(3'H)-furo[3,2-c]pyridine],
spiro[1-azabicyclo[2.2.2]octane-3,2'-(3'H)-furo[3,2-c]pyridine],
spiro[1-azabicyclo[2.2.2]octane-3,2'(3'H)-furo[2,3-b]pyridine-7'-oxide],
spiro[1-azabicyclo[2.2.2]octane-3,2'(3'H)-furo[2,3-b]pyridine-6'-carbonitrile],
6'-chlorospiro[1-azabicyclo[2.2.2]octane-3,2(3'H)-furo[2,3-b]pyridine],
6'-fluorospuro[1-azabicyclo[2.2.2]octane-3,2'(3'H)-furo[2,3-b]pyridine],
*N*-(1-azabicyclo[2.2.2]oct-3-yl)(5-phenylfuran-2-carboxamide),
*N*-(1-azabicyclo[2.2.2]oct-3-yl)(5-(3-fluorophenyl)furan-2-carboxamide),
*N*-(1-azabicyclo[2.2.2]oct-3-yl)(3-(3-thienyl)benzamide),
*N*-(1-azabicyclo[2.2-2]oct-3-yl)(3-phenylbenzamide),
*N*-(1-azabicyclo[2.2.2]oct-3-yl)(3-(3-pyridyl)benzamide),
*N*-(1-azabicyclo[2.2.2]oct-3-yl)(5-phenylthiophene-2-carboxamide),
*N-*(1-azabicyclo[2.2.2]oct-3-yl)(3-(3-methoxyphenyl)benzamide),
*N*-(1-azabicyclo[2.2.2]oct-3-yl)(3-(2-methoxyphenyl)benzamide),
*N*-(1-azabicyclo[2.2-2]oct-3-yl)(3-(3-(*N*-acetylamino)phenyl)benzamide),
*N*-(1-azabicyclo[2.2.2]oct-3-yl)(3-(3-fluorophenyl)benzamide),
*N*-(1-azabicyclo[2.2.2]oct-3-yl)(3-(3,methylphenyl)benzamide),
*N*-(1-azabicyclo[2.2.2]oct-3-yl)(3-(2-thienyl)benzamide),
*N*-(1-azabicyclo[2.2.2]oct-3-yl)(3-(3,5-dichlorophenyl)benzamide),
*N*-(1-azabicyclo[2.2.2]oct-3-yl)(3-(2-naphthyl)benzamide),
*N*-(1-azabicyclo[2.2.2]oct-3-yl)(3-(4-fluorophenyl)benzamide),
*N*-(1-azabicyclo[2.2.2]oct-3-yl)(5-(3-pyridyl)furan-2-carboxamide),
*N*-(1-azabicyclo[2.2.2]oct-3-yl)(5-(3-thienyl)furan-2-carboxamide),
*N-*(1-azabicyclo[2.2.2]oct-3-yl)(5 -(2-benzo[b]furanyl)furan-2-carboxamide),
*N*-(1-azabicyclo[2.2.2]oct-3-yl)(5-(4-pyridyl)furan-2-carboxamide),
*N*-(1-azabicyclo[2.2.2]oct-3-yl)(5-(2-thienyl)furan-2-carboxamide),
*N*-(1-azabicyclo[2.2.2]oct-3-yl)(5-(3-methoxyphenyl)furan-2-carboxamide),
*N*-(1-azabicyclo[2.2.2]oct-3-yl)(5-(2-methoxyphenyl)furan-2-carboxamide),
*N*-(1-azabicyclo[2.2.2]oct-3-yl)(5-(4-fluorophmyl)faran-2-carboxamide),
*N*-(1-azabicyclo[2.2.2]oct-3-yl)(5-(2-naphthyl)furan-2-carboxamide),
*N*-(1-azabicyclo[2.2.2]oct-3-yl)(5-(3-methylphenyl)furan-2-carboxamide),
*N*-(1-azabicyclo[2.2.2]oct-3-yl)(5-(3-furyl)furan-2-carboxamide),
*N*-(1-azabicyclo[2.2.2]oct-3-yl)(5-(2-furyl)furan-2-carboxamide),
*N*-(1-azabicyclo[2.2.2]oct-3-yl)(5-(2-pyridyl)fura-2-carboxamide),
*N*-(1-azabicyclo[2.2.2]oct-3-yl)(5-(4-pyridyl)thiophene-2-carbaxamide),
*N*-(1-azabicyclo[2.2.2]oct-3-yl)(5-(3-pyridyl)thiophene-2-carboxamide).
*N*-(1-azabicyclo[2.2.2]oct-3-yl)(5-(2-pyridyl)thiophene-2-carboxamide),
*N*-(1-azabicyclo[2.2.2]oct-3-yl)(4-(2-pyidyl)thiophene-2-carboxamide),
*N*-(1-azabicyclo[2.2.2]oct-3-yl)(4-(4-pyridyl)thiophene-2-carboxamide),
*N*-(1-azabicyclo[2.2.2]oct-3-yl)(4-(3-pyridyl)thiophene-2-carboxamide),
*N-*(1-azabicyclo[2.2.2]oct-3-yl)(5-(3-(*N*-acetylamino)phenyl)furan-2-carboxamide),
*N*-(1-azabicyclo[2.2.2]oct-3-yl)(5-(3-nitrophenyl)furan-2-carboxamide),
*N*-(1-azabicyclo[2.2.2]oct-3-yl)(5-(3-trifluoromethylphenyl)furan-2-carboxamide),
*N*-(1-azabicyclo[2.2.2]oct-3-yl)(5-(3-chlorophenyl)furan-2-carboxamide),
*N*-(1-azabicyclo[2.2.2]oct-3-yl)(5-(3-(*N*-acetylamino)phenyl)thiophene-2-carboxamide),
*N*-(1-azabicyclo[2,2.2]oct-3-yl)(5-(3-fluorophenyl)thiophene-2-carboxamide),
*N*-(1-azabicyelo[2.2.2]oct-3-yl)(5-(3-methoxyphenyl)thiophene-2-carboxamide),
*N*-(1-azabicyclo[2.2.2]oct-3-yl)(5-(3-ethoxyphenyl)thiophene-2-carboxamide),
*N*-(1-azabicyclo[2.2.2]oct-3-yl)(5-(3,5-dimethylisoxazol-4-yl)furan-2-carboxamide),
*N*-(1-azabicyclo[2.2.2]oct-3-yl)(5-(3,5-dimethylisoxazol-4-yl)thiophene-2-carboxamide),
*N*-(1-azabicyclo[2.2.2]oct-3-yl)(5-(3-aminophenyl)thiophene-2-carboxamide),
*N*-(1-azabicyclo[2.2.2]oct-3-yl)(5-(3-pyridyl)thiophene-3-carboxamide),
*N*-(1-azabicyclo[2.2.2]oct-3-yl)(5-(4-ehlorophenyl)furan-2-carboxamide),
*N*-(1-azabicyclo[2.2.2]oct-3-yl)(5-(3-pyridyl)thiazole-3-carboxamide),
*N*-(1-azabicyclo[2-2.2]oct-3-yl)(5-(4-pyridyl)thiazole-3-carboxamide),
*N*-(1-azabicyclo[2.2.2]oct-3-yl)(5-(3-(*N*,*N*-dimethylamino)phenyl)thiophene-2-carboxamide),
*N*-(1-azabicyclo[2.2.2]oct-3-yl)(5-(8-quinolinyl)thiophene-2-carboxamide),
*N*-(1-azabicyclo[2.2.2]oct-3-yl)(5-phenylthiophene-3-carboxamide),
*N-*(1-azabicyclo[2.2.2]oct-3-yl)(4-phenylthiophene-2-carboxamide),
*N*-(1-azabicyclo[2.2.2]oct-3-yl)(5-(3-cyanophenyl)thiophene-2-carboxamide),
*N*-(1-azabicyclo[2.2.2]oct-3-yl)(5-(3-(*N*-methylamino)phenyl)thiophene-7-carboxamide),
*N*-(1-azabicyclo[2.2.2]oct-3-yl)(5-(3-hydroxyphenyl)thiophene-2-carboxamide),
*N*-(1-azabicyclo[2.2.2]oct-3-yl)(5-(3-pyridylamino)thiophene-2-carboxamide),
*N*-(1-azabicyclo[2.2.2]oct-3-yl)(5-(3-chlorophenyl)thiophene-2-carboxamide),
*N*-(1-aza-bicyclo[2.2.2]oct-3-yl)(5-(3-(4-morpholinyl)phenyl)thiophene-2-carboxamide),
*N*-(1-azabicyclo[2.2.2]oct-3-yl)(5-(3-(aminomethyl)phenyl)thiophene-2-carboxamide),
*N*-(1-azabieyclo[2.2.2]act-3-yl)(5-phenoxythiaphene-2-carboxamide),
*N*-(1-azabicyclo[2.2.2]oct-3-yl)(5-(3-aminophenyl)furan-2-carboxamide),
*N*-(1-azabicyclo[2.2.2]oct-3-yl)(5-(3-(*N*,*N*-dimethylamino)phenyl)furan-2-carboxamide),
*N*-(1-azabicyclo[2.2.2]oct-3-yl)(5-(3-formylphenyl)thiophene-2-carboxamide); *N-*(1-azabicyclo[2.2.2]oct-3-yl)(5-(3-(hydroxymethyl)phenyl)thiophene-2-carboxamide),
(*R*)-*N*-(1-azabicyclo[2.2.2]oct-3-yl)(5-phenylfuran-2-carboxamide),
*(R)-N*-(1-azabicyclo[2.2.2]oct-3-yl)(5-(3-fluorophenyl)furan-2-carboxamide),
*(R)-N*-(1-azabicyclo[2.2.2]oct-3-yl)(3-(3-thienyl)benzamide),
*(R)*-*N*-(1-azabicyclo[2.2.2]oct-3-yl)(3-phenylbenzamide),
*(R)*-*N*-(1-azabicyclo[2.2.2]oct-3-yl)(3-(3-pyridyl)benzamide),
*(R)*-*N*-(1-azabicyclo[2.2.2]oct-3-yl)(5-phenylthiophene-2-carboxamide),
*(R)*-*N*-(1-azabicyclo[2.2.2]oct-3-yl)(3-(3-methoxyphenyl) benzamide),
*(R)*-*N*-(1-azabicyclo[2.2.2]oct-3-yl)(3-(2-methoxyphenyl)benzamide),
*(R)*-*N*-(1-azabicyclo[2.2.2]oct-3-yl)(3-(3-(*N*-acetylamino)phenyl)benzamide);
*(R)-N*-(1-azabicyclo[2.2.2]oct-3-yl)(3-(3-fluoroplzenyl)benzamide),
(*R*)-*N*-(1-azabicyclo[2.2.2]oct-3-yl)(3-(3-methylphenyl)benzamide),
(*R*)-*N*-(1-azabicyclo[2.2.2]oct-3-yl)(3-(2-thienyl)benzamide),
(*R*)-*N*-(1-azabicyclo[2.2.2]oct-3-yl)(3-(3,5-dichlorophenyl)benzamide),
(*R*)-*N*-(1-azabicyclo[2.2.2]oct-3-yl)(3-(2-naphthyl)benzamide),
(*R*)-*N*-(1-azabicyclo[2.2.2]oct-3-yl)(3-(4-fluorophenyl)benzamide),
(*R*)-*N*-(1-azabicyclo[2.2.2]oct-3-yl)(5-(3-pyridyl)furan-2-carboxamide),
(*R*)-*N*-(1-azabicyclo[2.2.2]oct-3-yl)(5-(3-thienyl)furan-carboxamide),
(*R*)-*N*-(1-azabicyclo[2.2.2]oct-3-yl)(5-(2-benzo[b]furanyl)furan-2-carboxamide),
(*R*)-*N*-(1-azabicyclo[2.2.2]oct-3-yl)(5-(4-pyridyl)furan-2-carboxamide),
(*R*)-*N*-(1-azabicyclo[2.2.2]oct-3-yl)(5-(2-thienyl)furan-2-carboxamide),
*(R)*-*N*-(1-azabicyclo[2.2.2]oct-3-yl)(5-(3-methoxyphenyl)furan-2-carboxamide),
(*R*)-*N*-(1-azabicyclo[2.2.2]oct-3-yl)(5-(2-methoxyphenyl)furan-2-carboxamide),
*(R)*-*N*-(1-azabicyclo[2.2.2]oct-3-yl)(5-(4-fluorophenyl)furan-2-carboxamide),
*(R)-N-*(1-azabicyclo[2.2.2]oct-3-yl)(5-(2-naphthyl)furan-2-carboxamide),
*(R)-N-*(1-azabicyclo[2.2.2]oct-3-yl)(5-(3-methylphenyl)furan-2-carboxamide),
*(R)-N*-(1-azabicyclo[2.2.2]oct-3-yl)(5-(3-furyl)furan-2-carboxamide),
*(R)-N*-(1-azabicyclo[2.2.2]oct-3-yl)(5-(2-funyl)furan-2-carboxamide),
*(R)*-*N* (1-azabicyclo[2.2.2]oct-3-yl)(5-(2-pyridyl)furan-2-carboxamide),
*(R)-N*-(1-azabicyclo[2.2.2]oct-3-yl)(5-(4-pyridyl)thiophene-2-carboxamide),
*(R)-N*-(1-azabicyclo[2.2.2]oct-3-yl)(5-(3-pyridyl)thiophene-2-carboxamide),
*(R)-N*-(1-azabicyclo[2.2.2]oct-3-yl)(5-(2-pyridyl)thiophene-2-carboxamide),
*(R)-N-*(1-azabicyclo[2.2.2]oct-3-yl)(4-(2-pyridyl)thiophene-2-carboxamide),
*(R)-N*-(1-azabicyclo[2.2.2]oct-3-yl)(4-(4-pyridyl)thiophene-2-carboxamide),
*(R)*-*N-*(1-azabicyclo[2.2.2]oct-3-yl)(4-(3-pyridyl)thiophene-2-carboxamide),
*(R)-N*-(1-azabicyclo[2.2.2]oct-3-yl)(5-(3-(N acetylamino)phenyl)furan-2-carboxamide),
*(R)-N-*(1-azabicyclo[2.2.2]oct-3-yl)(5-(3-nitrophenyl)furan-2-carboxamide),
*(R)-N*-(1-azabicyclo[2.2.2]oct-3-yl)(5-(3-trifluoromethylphenyl)furan-2-carboxamide),
*(R)-N-*(1-azabicyclo[2.2.2]oct-3-yl)(5-(3-chlorophenyl)furan-2-carboxamide),
*(R)-N*-(1-azabicyclo[2.2.2]oct-3-yl)(5-(3-(*N*-acetylamino)phenyl)thiophene-2-carboxamide),
*(R)-N-(*1-azabicyclo[2.2.2]oct-3-yl)(5-(3-fluorophenyl)thiophene-2-carboxamide),
*(R)-N-*(1-azabicyclo[2.2.2]oct-3-yl)(5-(3-methoxyphenyl)miophene-2-carboxamide),
*(R)-N*-(1-azabicyclo[2.2.2]oct-3-yl)(5-(3-ethoxyphenyl)thiophene-2-carboxamide),
*(R)-N-*(1-azabicyclo[2.2.2]oct-3-yl)(5-(3,5-dimethylisoxazol-4-yl)furan-2-carboxamide),
*(R)-N*-(1-azabicyclo[2.2.2]oct-3-yl)(5-(3,5-dimethylisoxazol-4-yl)thiophene-2-carboxamide),
*(R)-N-*(1-azabicyclo[2.2.2]oct-3-yl)(5-(3-aminophenyl)thiophezze-2-carboxamide),
*(R)-N-*(1-azabicyclo[2.2.2]oct-3-yl)(5-(3-pyridyl)thiophene-3-carboxamide),
*(R)-N*-(1-azabicyclo[2.2.2]oct-3-yl)[5-(4-chlorophenyl)furan-2-carboxamide),
*(R)*-*N*-(1-azabicyclo[2.2.2]oct-3-yl)(5-(3-pyridyl)thiazole-3-carboxamide),
*(R)*-*N*-(1-azabicyclo[2.2.2]oct-3-yl)(5-pyridyl)thiazole-3-carboxamide),
*(R)*-*N*-(1-azabicyclo[2.2.2]oct-3-yl)(5-(3-(*N,N*-dimethylamino)phenyl)thiophene-2-carboxamide),
*(R)*-*N*-(1-azabicyclo[2.2.2]oct-3-yl)(5-(8-quinolinyl)thiophene-2-carboxamide),
*(S)*-*N*-(1-azabicyclo[2.2.2]oct-3-yl)(5-(3-pyridyl)thiophene-2-carboxamide);
*(S)-N*-(1-azabicyclo[2.2.2]oct-3-yl)(5-(4-pyridyl)thiophene-2-carboxamide),
*(S)*-*N*-(1-azabicyclo[2.2.2]oct-3 -yl)(5-(2-pyridyl)thiophene-2-carboxamide),
*(S)*-*N*-(1-azabicyclo[2.2.2]oct-3-yl)(5-phenylthiophene-2-carboxamide),
*(R)*-*N*(1-azabicyclo[2.2.2]oct-3-yl)(5-phenylthiophene-3-carboxamide),
*(R)*-*N*-(1-azabicyclo[2.2.2]oct-3-yl)(4-phenylthiophene-2-carboxamide),
*(R)*-*N*-(1-azabicyclo[2.2.2]oct-3-yl)(5-(3-cyanophenyl)thiophene-2-carboxamide),
*(R)*-*N*-(1-azabicyclo[2.2.2]oct-3-yl)(5-(3-(*N*-methylamino)phenyl)thiophene-2-carboxamide),
(*R*)-*N*-(1-aza-bicyclo[2.2.2]oct-3-yl)(5-(3-hydroxyphenyl)thiophene-2-carboxamide),
(*R*)-*N*-(1-azabicyclo[2.2.2]oct-3-yl)(5-(3-pyridylamino)thiophene-2-carboxamide),
(*R*)-*N*-(1-azabicyclo[2.2.2]oct-3-yl)(5-(3-chlorophenyl)thiophene-2-carbaxamide),
(*R*)-*N*-(1-azabicyclo[2.2.2]oct-3-yl)(5-(3-(4-morpholinyl)phenyl)thiophene-2-carboxamide),
(*R*)-*N*-(1-azabicyclo[2.2.2]oct-3-yl)(5-(3-(aminomethyl)phenyl)thiophene-2-carboxamide),
(*R*)-N-(1-azabicyclo[2.2.2]oct-3-yl)(5-phenoxythiophene-2-carboxamide),
(*R*)-*N*-(1-azabicyclo[2.2.2]oct-3-yl)(5-(3-aminophenyl)furan-2-carboxamide).
*(R)*-*N*-(1-azabicyclo[2.2.2]oct-3-yl)(5-(3-(*N,N*-dimethylamino)phenyl)furan-2-carboxamide),
*(R)*-*N*-(1-azabicyclo[2.2.2]oct-3-yl)(5-(3-formylphenyl)thiophene-2-carboxamide), or
(*R*)-*N*-(1-azabicyclo[2.2.2]oct-3-yl)(5-(3-(hydroxymethyl)phenyl)thiophene-2-carboxamide), or a pharmaceutically-acceptable salt thereof.

2. A pharmaceutical composition comprising a combination according to Claim 1 together with a pharmaceutically acceptable diluent or carrier.

3. The use of a combination according to Claim 1 in the preparation of a medicament providing neuroprotection or analgesia for the treatment or prophylaxis of a condition or disorder involving reduced cholinergic function selected from Alzheimer's disease, cognitive or attention disorders, anxiety, depression, smoking cessation, schizophrenia, Tourette's syndrome, and Parkinson's disease.

4. The use of a combination according to Claim 3, in the preparation of a medicament for the treatment or prophylaxis of Alzheimer's disease.

5. A combination according to Claim 1, wherein said statin is rosuvastatin or a pharmaceutically-acceptable salt thereof and
said α7-nAChR agonist is selected from:
spiro[1-azabicyclo[2.2.2]octane-3,5'-oxazolidine]-2'-one;
*N*-(1-Azabicyclo[2.2.2]oct-3-yl)[*E*-3-(2-thienyl)propenamide], or
(2'R)-5'-(3-furanyl)spiro[1-azabicyclo[2.2.2]octane-3,2'-(3'H)-furo[2,3-b]pyridine] or a pharmaceutically-acceptable salt thereof.

6. A pharmaceutical composition comprising a combination according to Claim 5 together with a pharmaceutically acceptable diluent or carrier.

7. The use of a combination according to Claim 5, in the preparation of a medicament providing neuroprotection or analgesia for the treatment or prophylaxis of a condition or disorder involving reduced cholinergic function selected from Alzheimer's disease, cognitive or attention disorders, anxiety, depression, smoking cessation, schizophrenia, Tourette's syndrome, and Parkinson's disease.

8. The use of a combination according to Claim 7, in the preparation of a medicament for the treatment or prophylaxis of Alzheimer's disease.

## Patentansprüche

1. Kombination, enthaltend:
ein Statin ausgewählt aus Atorvastatin, Cerivastatin, Fluvastatin, Lovastatin, Pravastatin-Natrium, Simvastatin und Rosuvastatin und deren pharmazeutisch annehmbare Salze und
einen α7-nAChR-Agonisten ausgewählt aus Spiro[1-azabicyclo[2.2.2]octan-3,5'-oxazolidin]-2'-on,
(+)Spiro[1-azabicyclo[2.2.2]octan-3,5'-oxazolidin]-2'-on,
(-)Spiro[1-azabicyclo[2.2.2]octan-3,5'-oxazolidin]-2'-on,
Spiro[1-azabicyclo[2.2.1]heptan-3,5'-oxazolidin-2'-on],
3'-Methylspiro[1-azabicyclo[2.2.2]octan-3,5'-oxazolidin-2'-on],
Spiro[1-azabicyclo[2.2.2]actan-3,2'-(3'H) - furo[2,3-b]pyridin],
5' Bromspiro[1-azabicyclo[2.2.2]octan-3,2'-(3'H)-furo[2,3-b]pyridin],
5'Phenylspiro[1-azabicyclo[2.2.2]octan-3,2'-(3'H)-furo[2,3-b]pyridin],
5'Nitrospira[1-azabicyclo[2.2.2]octan-3,2'-(3'H)-furo[2,3-b]pyridin],
1'Chlorspiro[1-azabicyclo[2.2.2]octan-3,2'-(3'H)-furo[2,3-b]isochinolin],
5'(Phenylcarbonsäureamid)spiro[1-azabicyclo[2.2.2]octan-3,2'-(3'H)-furo[2,3-b]pyridin],
5'(Phenylamincarbonylamin)spiro[1-azabicyclo[2.2.2]octan-3,2'-(3'H)-furo[2,3-b]pyridin],
5'(Phenylsulfonylamid)spiro[1-azabicyclo[2.2.2]octan-3,2'-(3'H)-furo[2,3-b] pyridin],
5'Aminspiro[1-azabicyclo[2.2.2]octan-3,2'-(3'H)-furo[2,3-b]pyridin],
5'-N-Methylaminspiro[1-azabicyclo[2.2.2]octan-3,2'-(3'H)-furo[2,3-b]pyridin],
5'-N,N-Dimethylaminspiro[1-azabicyclo[2.2.2]octan-3,2'-(3'H)-furo[2,3-b]pyridin],
5'-N,N-Diethylaminspiro[1-azabicyclo[2.2.2]octan-3,2'-(3'H)-furo[2,3-b]pyridin],
5'-N-Ethylaminspiro[1-azabicyclo[2.2.2]octan-3,2'-(3'H)-furo[2,3-b]pyridin],
5'-N-Benzylaminspiro[1-azabicyclo[2.2.2]octan-3,2'-(3'H)-furo[2,3-b]pyridin],
5'-N-Formamidspiro[1-azabicyclo[2.2.2]octan-3,2'-(3'H)-furo[2,3-b]pyridin],
5'-N-Acetamidspiro[1-azabicyclo[2.2.2]octan-3,2'-(3'H)-furo[2,3-b]pyridin],
Spiro[1-azabicyclo[2.2.2]octan-3,2'-(3'H)-furo[2,3-b]isochinolin],
Spiro[1-azabicyclo[2.2.2]octan-3,2'-(3'H)-furo[2,3-b]chinolin],
5'-Ethenylspiro[1-azabicyclo[2.2.2]octan-3,2'-(3'H)-furo [2,3-b]pyridin]
5'-(E)(Phenylethenyl)spiro[1-azabicyclo[2.2.2]octan-3,2'-(3'H)-furo[2,3-b]pyridin],
5'-(4-Morpholin)spiro[1-azabicyclo[2.2.2]octan-3,2'-(3'H)-furo[2,3-b]pyridin],
5'-(1-Azetidinyl)spiro[1-azabicyclo[2.2.2]octan-3,2'-(3'H)-furo[2,3-b]pyridin],
5'-(E)-(2-(4-Pyridyl)ethenyl)spiro[1-azabicyclo[2.2.2]octan-3,2'-(3'H)-furo[2,3-b]pyridin],
5'-(E)-(2-(2-Pyridyl)ethenyl)spiro[1-azabicyclo[2.2.2]octan-3,2'-(3'H)-furo[2,3-b]pyridin],
5'-(2-Trimethylsilylethenyl)spiro[1-azabicyclo[2.2.2]octan-3,2'-(3'H)-furo[2,3-b]pyridin],
5'-Ethenylspiro[1-azabicyclo[2.2.2]octan-3,2'-(3'H)-furo[2,3-b]pyridin],
5'-(2-Furyl)spiro[1-azabicyclo[2.2.2]octan-3,2'-(3'H)-furo[2,3-b]pyridin],
5'-(3-Pyridyl)spiro[1-azabicyclo[2.2.2]octan-3,2'-(3'H)-Euro[2,3-b]pyridin],
5'-Methylspiro[1-azabicyclo[2.2.2]octan-3,2'-(3'H)-furo[2,3-b]pyridin],
Spiro[1-azabicyclo[2.2.2]octan-3,2'-(3'H)-furo[2,3-b]pyridin-5'carbonsäurenitril],
Spiro[1-azabicyclo[2.2.2]octan-3,2'-(3'H)- furo[2,3-b]pyridin-5'carbonsäureamid],
5'-N' (3-Chlorphenyl)amincarbonylaminspiro[1-azabicyclo[2.2.2]octan-3,2'-(3'H)-furo[2,3-b]pyridin],
5'-N'(2-Nitrophenyl)amincarbonylaminspiro[1-azabicyclo[2.2.2]octan-3,2'-(3'H)-furo[2,3-b]pyridin],
4'-Chlorspiro[1-azabicyclo[2.2.2]octan-3,2'-(3'H)-furo [2, 3-b] pyridin],
4'-Methoxyspiro[1-azabicyclo[2.2.2]octan-3,2'-(3'H)-furo[2,3-b]pyridin],
4'-Phenylthiospiro[1-azabicyclo[2.2.2]octan-3,2'-(3'H)-furo[2,3-b]pyridin],
4'-(N-2-Aminoethyl)aminspiro[1-azabicyclo[2.2.2]octan-3,2'-(3'H)-furo[2,3-b]pyridin],
4'-Phenylaminspiro[1-azabicyclo[2.2.2]octan-3,2'-(3'H)-furo[2,3-b]pyridin],
4'-Methylaminspiro[1-azabicyclo[2.2.2]octan-3,2'-(3'H)-furo[2,3-b]pyridin],
4'-(4-N-Methylpiperazin-1-yl)spiro[1-azabicyclo[2.2.2]octan-3,2'-(3'H)-furo[2,3-b]pyridin],
4'-Chlorspiro[1-azabicyclo[2.2.2]octan-3,2'-(3'H)-furo[3,2-c]pyridin],
Spiro[1-azabicyclo[2.2.2]octan-3,2'-(3'H)-furo[3,2-c]pyridin],
Spiro[1-azabicyclo[2.2.2]octan-3,2'-(3'H)-furo[2,3-b]pyridin-7'-oxid],
Spiro[1-azabicyclo[2.2.2]octan-3,2'(3'H)-furo[2,3-b]pyridin-6'-karbsonsäurenitril],
6'-Chlorspiro[1-azabicyclo[2.2.2]octan-3,2'(3'H)-furo[2,3-b]pyridin],
6'-Fluorspiro[1-azabicyclo[2.2.2]octan-3,2' (3'H)-furo[2,3-b]pyridin],
N-(1-Azabicyclo[2.2.2]oct-3-yl)(5-phenylfuran-2-carbonsäureamid),
N-(1-Azabicyclo[2.2.2]oct-3-yl)(5-(3-fluorphenyl)furan-2-carbonsäureamid),
N-(1-Azabicyclo[2.2.2]oct-3-yl)(3-(3-thienyl)benzamid),
N-(1-Azabicyclo[2.2.2]oct-3-yl)(3-phenylbenzamid),
N-(1-Azabicyclo[2.2.2]oct-3-yl)(3-(3-pyridyl)benzamid),
N-(1-Azabicyclo[2.2.2]oct-3-yl)(5-phenylthiophen-2-carbonsäureamid),
N-(1-Azabicyclo[2.2.2]oct-3-yl)(3-(3-methoxyphenyl)benzamid),
N- (1-Azabicyclo [2 .2 . 2] oct-3-yl) (3- (2-methoxyphenyl)benzamid),
N- (1-Azabicyclo [2 .2 .2] oct-3-yl) (3- (3- (N-acetylamin)phenyl)benzamid),
N-(1-Azabicyclo[2.2.2]oct-3-yl)(3-(3-fluorphenyl)benzamid),
N- (1-Azabicyclo [2 .2 . 2] oct-3-yl) (3- (3-methylphenyl)benzamid),
N-(1-Azabicyclo[2.2.2]oct-3-yl)(3-(2-thienyl)benzamid),
N-(1-Azabicyclo[2.2.2]oct-3-yl)(3-(3,5-dichlorphenyl)benzamid),
N-(1-Azabicyclo[2.2.2]oct-3-yl)(3-(2-naphthyl)benzamid),
N-(1-Azabicyclo[2.2.2]oct-3-yl)(3-(,l-fluorphenyl)benzamid),
N-(1-Azabicyclo[2.2.2]oct-3-yl)(5-(3-pyridyl)furan-2-carbonsäureamid),
N-(1-Azabicyclo[2.2.2]oct-3-yl)(5-(3-thienyl)furan-2-carbonsäureamid),
N-(1-Azabicyclo[2.2.2]oct-3-yl)(5-(2-benzo[b]furanyl)furan-2-carbonsäureamid),
N-(1-Azabicyclo[2.2.2]oct-3-yl)(5-(4-pyridyl)furan-2-carbonsäureamid),
N-(1-Azabicyclo[2.2.2]oct-3-yl)(5-(2-thienyl)furan-2-carbonsäureamid),
N-(1-Azabicyclo[2.2.2]oct-3-yl)(5-(3-methoxyphenyl)furan-2-carbonsäureamid),
N-(1-Azabicyclo[2.2.2]oct-3-yl)(5-(2-methoxyphenyl)furan-2-carbonsäureamid),
N-(1-Azabicyclo[2.2.2]oct-3-yl)(5-(4-fluorphenyl)furan-2-carbonsäureamid),
N-(1-Azabicyclo[2.2.2]oct-3-yl)(5-(2-naphthyl)furan-2-carbonsäureamid),
N-(1-Azabicyclo[2.2.2]oct-3-yl)(5-(3-methylphenyl)furan-2-carbonsäureamid),
N-(1-Azabicyclo[2.2.2]oct-3-yl)(5-(3-furyl)furan-2-carbonsäureamid),
N-(1-Azabicyclo[2.2.2]oct-3-yl)(5-(2-furyl)furan-2-carbonsäureamid),
N-(1-Azabicyclo[2.2.2]oct-3-yl)(5-(2-pyridyl)furan-2-carbonsäureamid),
N-(1-Azabicyclo[2.2.2]oct-3-yl)(5-(4-pyridyl)thiophen-2-carbonsäureamid),
N-(1-Azabicyclo[2.2.2]oct-3-yl)(5-(3-pyridyl)thiophen-2-carbonsäureamid),
N-(1-Azabicyclo[2.2.2] oct-3-yl)(5-(2-pyridyl)thiophen-2-carbonsäureamid),
N-(1-Azabicyclo[2.2.2]oct-3-yl)(4-(2-pyridyl)thiophen-2-carbonsäureamid),
N-(1-Azabicyclo[2.2.2] oct-3-yl)(4-(4-pyridyl)thiophen-2-carbonsäureamid),
N-(1-Azabicyclo[2.2.2]oct-3-yl)(4-(3-pyridyl)thiophen-2-carbonsäureamid),
N-(1-Azabicyclo[2.2.2]oct-3-yl)(5-(3-(N-acetylamin)phenyl)furan-2-carbonsäureamid),
N-(1-Azabicyclo[2.2.2]oct-3-yl)(5-(3-nitrophenyl)furan-2-carbonsäureamid),
N-(1-Azabicyclo[2.2.2]oct-3-yl)(5-(3-trifluormethylphenyl)furan-2-carbonsäureamid),
N-(1-Azabicyclo[2.2.2]oct-3-yl)(5-(3-chlorphenyl)furan-2-carbonsäureamid),
N-(1-Azabicyclo[2.2.2]oct-3-yl)(5-(3-(N-acetylamin)phenyl)thiophen-2-carbonsäureamid),
N-(1-Azabicyclo[2.2.2]oct-3-yl)(5-(3-fluorphenyl)thiophen-2-carbonsäureamid),
N- (1-Azabicyclo [2.2.2] oct-3-yl) (5- (3-methoxyphenyl)thiophen-2-carbonsäureamid),
N-(1-Azabicyclo[2.2.2]oct-3-y1)(5-(3-ethoxyphenyl)thiophen-2-carbansäureamid),
N- (1-Azabicyclo [2 .2 .2] oct-3-yl) (5- (3,5-dimethylisoxazol-4-yl)furan-2-carbonsäureami.d),
N-(1-Azabicyclo[2.2.2]oct-3-yl)(5-(3,5-dimethylisoxazol-4-yl)thiophen-2-carbonsäureamid),
N-(1-Azabicyclo[2.2.2]oct-3-yl)(5-(3-aminophenyl)thiophen-2-carbonsäureamid),
N-(1-Azabicyclo[2.2.2]oct-3-yl)(5-(3-pyridyl)thiophen-3-carbonsäureamid),
N-(1-Azabicyclo[2.2.2]oct-3-yl)(5-(4-chlorphenyl)furan-2-carbonsäureamid),
N-(1-Azabicyclo[2.2.2]oct-3-yl)(5-(3-pyridyl)thiazol-3-carbonsäureamid),
N-(1-Azabicyclo[2.2.2]oct-3-yl)(5-(4-pyridyl)thiazol-3-carbonsäureamid),
N-(1-Azabicyclo[2.2.2]oct-3-yl)(5-(3-(N,N-dimethylamin)phenyl)thiophen-2-carbonsäureamid),
N-(1-Azabicyclo[2.2.2]oct-3-yl)(5-(8-chinolinyl)thiophen-2-carbonsäureamid),
N-(1-Azabicyclo[2.2.2]oct-3-yl) (5-phenylthiophen-3-carbonsäureamid),
N-(1-Azabicyclo[2.2.2]oct-3-yl)(4-phenylthiophen-2-carbonsäureamid),
N-(1-Azabicyclo[2.2.2]octw3-yl)(5-(3-cyanphenyl)thiophen-2-carbonsäureamid),
N-(1-Azabicyclo[2.2.2]oct-3-yl)(5-(3-(N-methylamin)phenyl)thiophen-2-carbonsäureamid),
N-(1-Azabicyclo[2.2.2]oct-3-yl)(5-(3-hydroxyphenyl)thiophen-2-carbonsäureamid),
N-(1-Azabicyclo[2.2.2]oct-3-yl)(5-(3-pyridylamin)thiophen-2-carbonsäureamid),
N-(1-Azabicyclo[2.2.2]oct-3-yl)(5-(3-chlorphenyl)thiophen-2-carbonsäureamid),
N-(1-Azabicyclo[2.2.2]oct-3-yl)(5-(3-(4-morpholinyl)phenyl)thiophen-2-carbonsäureamid),
N-(1-Azabicyclo[2.2.2]oct-3-yl)(5-(3-(aminomethyl)phenyl)thiophen-2-carbonsäureamid),
N-(1-Azabicyclo[2.2.2]oct-3-yl)(5-phenoxythiophen-2-carbonsäureamid),
N-(1-Azabicyclo[2.2.2]oct-3-yl)(5-(3-aminophenyl)furan-2-carbonsäureamid),
N-(1-Azabicyclo[2.2.2]oct-3-yl)(5-(3-(N,N-dimethylamin)phenyl)furan-2-carbonsäureamid),
N-(1-Azabicyclo[2.2.2]oct-3-yl)(5-(3-formylphenyl)thiophen-2-carbonsäureamid),
N-(1-Azabicyclo [2.2.2]oct-3-yl)(5-(3-(hydroxymethyl)phenyl)thiophen-2-carbonsäureamid),
(R)-N-(1-Azabicyclo[2.2.2]oct-3-yl)(5-phenylfuran-2-carbonsäureamid),
(R)-N-(1-Azabicyclo[2.2.2]oct-3-yl)(5-(3-fluorphenyl)furan-2-carbonsäureamid),
(R)-N-(1-Azabicyclo[2.2.2]oct-3-yl)(3-(3-thienyl)benzamid),
(R)-N-(1-Azabicyclo[2.2.2]oct-3-yl)(3-phenylbenzamid),
(R)-N-(1-Azabicyclo[2.2.2]oct-3-yl)(3-(3-pyridyl)benzamid),
(R)-N-(1-Azabicyclo[2.2.2]oct-3-yl)(5-phenylthiophen-2-carbonsäureamid),
(R)-N-(1-Azabicyclo[2.2.2]oct-3-yl)(3-(3-methoxyphenyl)benzamid),
(R)-N-(1-Azabicyclo[2.2.2]oct-3-yl)(3-(2-methoxyphenyl)benzamid),
(R)-N-(1-Azabicyclo[2.2.2]oct-3-yl)(3-(3-N-acetylamin)phenyl)benzamid),
(R)-N-(1-Azabicyclo[2.2.2]oct-3-yl)(3-(3-fluorphenyl)benzamid),
(R)-N-(1-Azabicyclo[2.2.2]oct-3-yl)(3-(2-thienyl)benzamid),
(R)-N-(1-Azabicyclo[2.2.2]oct-3-yl)(3-(3,5-dichlorphenyl)benzamid),
(R)-N-(1-Azabicyclo[2.2.2]oct-3-yl)(3-(2-naphthyl)benzamid),
(R)-N-(1-Azabicyclo[2.2.2]oct-3-yl)(3-(4-fluorphenyl)benzamid),
(R)-N-(1-Azabicyclo[2.2.2]oct-3-yl)(5-(3-pyridil)furan-2-carbonsäureamid),
(R)-N-(1-Azabicyclo[2.2.2]oct-3-yl)(5-(3-thienyl)furan-2-carbonsäureamid),
(R)-N-(1-Azabicyclo[2.2.2]oct-3-yl)(5-(2-benzo(b)furanyl)furan-2-carbonsäureamid),
(R)-N-(1-Azabicyclo[2.2.2]oct-3-yl)(5-(4-pyridyl)furan-2-carbonsäureamid),
(R)-N-(1-Azabicyclo [2 .2 .2] oct-3-yl) (5- (2-thienyl)furan-2-carbonsäureamid),
(R) -N- (1-Azabicyclo [2 .2.2] oct-3-yl) (5- (3-methoxyphenyl)furan-2-carbonsäureamid),
(R)-N-(1-Azabicyclo[2.2.2]oct-3-yl)(5-(2-methoxyphenyl)furan-2-carbonsäureamid),
(R)-N-(1-Azabicyclo[2.2.2]oct-3-yl)(5-(4-fluorphenyl)furan-2-carbonsäureamid),
(R)-N-(1-Azabicyclo[2.2.2]oct-3-yl) (5-(2-naphthyl)furan-2-carbonsäureamid),
(R) -N- (1-Azabicycla [2.2.2] oct-3-yl) (5-(3-methylphenhyl)furan-2-carborlsäureamid),
(R)-N-(1-Azabicyclo [2.2.2] oct-3-yl) (5-(3-furyl)furan-2-carbonsäureamid),
(R)-N-(1-Azabicyclo[2.2.2] oct-3-yl) (5-(2-furyl)furan-2-carbonsäureamid),
(R)-N-(1-Azabicyclo[2.2.2]oct-3-yl)(5-(2-pyridyl)furan-2-carbonsäureamid),
(R)-N-(1-Azabicyclo[2.2.2]oct-3-yl)(5-(4-pyridyl)thiophen-2-carbonsäureamid),
(R)-N-(1-Azabicyclo[2.2.2]oct-3-yl) (5- (3-pyridyl) thiophen-2-carbonsäureamid),
(R)-N-(1-Azabicyclo[2.2.2]oct-3-yl)(5-(2-pyridyl)thiophen-2-carbonsäureamid),
(R)-N-(1-Azabicyclo[2.2.2]oct-3-yl)(4-(2-pyridyl) thiophen-2-carbonsäureamid),
(R)-N-(1-Azabicyclo[2.2.2]oct-3-yl)(4-(4-pyridyl) thiophen-2-carbonsäureamid),
(R)-N-(1-Azabicyclo[2.2.2]oct-3-yl)(4-(3-pyridyl)thiophen-2-carbonsäureamid),
(R)-N-(1-Azabicyclo[2.2.2]oct-3-yl)(5-(3-(N-acetylamin)phenyl)furan-2-carbonsäureamid),
(R)-N-(1-Azabicyclo[2.2.2]oct-3-yl)(5-(3-nitrophenyl)furan-2-carbonsäureamid),
(R)-N-(1-Azabicyclo[2.2.2]oct-3-yl)(5-(3-trifluormethylphenyl)furan-2-carbonsäureamid),
(R)-N-(1-Azabicyclo[2.2.2]oct-3-yl)(5-(3-chlorophenyl)furan-2-carbonsäureamid),
(R)-N-(1-Azabieyclo[2.2.2]oct-3-yl)(5-(3-(N-acetylamin)phenyl)thiophen-2-carbansäureamid),
(R)-N-(1-Azabicyclo[2.2.2]oct-3-yl)(5-(3-fluorphenyl)thiophen-2-carbonsäureamid),
(R)-N-(1-Azabieyclo[2.2.2]oct-3-yl)(5-(3-methoxyphenyl)thiophen-2-carbonsäureamid),
(R)-N-(1-Azabicyclo[2.2.2]oct-3-yl)(5-(3-ethoxyphenyl)thiophen-2-carbonsäureamid),
(R)-N-(1-Azabicyclo[2.2.2]oct-3-yl)(5-(3,5-dimethylisoxazol-4-yl)furan-2-carbonsäureamid),
(R)-N-(1-Azabicyclo[2.2.2]oct-3-yl)(5-(3,5-dimethylisoxazol-4-yl)thiophen-2-carbonsäureamid),
(R)-N-(1-Azabicyclo[2.2.2]oct-3-yl)(5-(3-aminophenyl)thiophen-2-carbonsäureamid),
(R)-N-(1-Azabicyclo[2.2.2]oct-3-yl)(5-(3-pyridyl) thiophen-3-carbonsäureamid),
(R) -N- (1-Azabicyclo [2.2.2] oct-3-yl) (5- (4-chlorphenyl)furan-2-carbonsäureamid),
(R)-N-(1-Azabicyclo[2.2.2]oct-3-yl)(5-(3-pyridyl)thiazol-3-carbonsäureamid),
(R)-N-(1-Azabicyclo[2.2.2]oct-3-yl) (5-(4-pyridyl)thiazol-3-carbonsäureamid),
(R)-N-(1-Azabicyclo[2.2.2]oct-3-yl)(5-(3-(N,N-dimethylamin)phenyl)thiophen-2-carbonsäureamid),
(R)-N-(1-Azabicyclo[2.2.2] oct-3-yl)(5-(8-chinolinyl)thiophen-2-carbonsäureamid),
(S)-N-(1-Azabicyclo[2.2.2]oct-3-yl)(5-(3-pyridil)thiophen-2-carbonsäureamid),
(S)-N-(1-Azabicyclo[2.2.2]oct-3-yl)(5-(4-pyridil)thiophen-2-carbonsäureamid),
(S)-N-(1-Azabicyclo[2.2.2]oct-3-yl)(5-(2-pyridil)thiophen-2-carbonsäureamid),
(S)-N-(1-Azabicyclo[2.2.2]oct-3-y1)(5-phenylthiophen-2-carbonsäureamid),
(R)-N-(1-Azabicyclo[2.2.2]oct-3-yl)(5-phenylthiophen-3-carbonsäureamid),
(R)-N-(1-Azabicyclo[2.2.2]oct-3-yl)(4-phenylthiophen-2-carbonsäureamid),
(R)-N-(1-Azabicyclo[2.2.2]oct-3-yl) (5-(3-cyanphenyl)thiophen-2-carbonsäureamid),
(R)-N-(1-Azabicyclo[2.2.2]oct-3-yl)(5-(3-(N-methylamin)phenyl)thiophen-2-carbonsäureamid),
(R)-N-(1-Azabicyclo[2.2.2]oct-3-yl)(5-(3-hydroxyphenyl)thiophen-2-carbonsäureamid),
(R)-N-(1-Azabicyclo[2.2.2]oct-3-yl)(5-(3-pyridilamin)thiophen-2-carbonsäureamid),
(R)-N-(1-Azabicyclo[2.2.2]oct-3-yl)(5-(3-chlorphenyl)thiophen-2-carbonsäureamid),
(R)-N-(1-Azabicyclo[2.2.2]oct-3-yl)(5-(3-(4-morpholinyl)phenyl)thiophen-2-carbonsäureamid),
(R)-N-(1-Azabicyclo[2.2.2]oct-3-yl)(5-(3-(aminomethyl)phenyl)thiophen-2-carbonsäureamid),
(R)-N-(1-Azabicyclo[2.2.2]oct-3-yl)(5-phenoxythiophen-2-carbonsäureamid),
(R)-N-(1-Azabicyclo[2.2.2]oct-3-yl)(5-(3-aminophenyl)furan-2-carbonsäureamid),
(R)-N-(1-Azabicyclo[2.2.2]oct-3-yl)(5-(3-(N,N-dimethylamin)phenyl)furan-2-carbansäureamid),
(R)-N-(1-Azabicyclo[2.2.2]oct-3-yl)(5-(3-formylphenyl)thiophen-2-carbonsäureamid), oder
(R)-N-(1-Azabicyclo[2.2.2]oct-3-yl)(5-(3-hydroxymethyl)phenyl)thiophen-2-carbonsäureamid),
und deren pharmazeutisch annehmbare Salze.

2. Pharmazeutische Zusammensetzung, enthaltend eine Kombination nach Anspruch 1 zusammen mit einem pharmazeutisch annehmbaren Verdünnungsmittel oder Träger.

3. Verwendung einer Kombination nach Anspruch 1 bei der Herstellung eines Medikaments, das neuroprotektiv oder analgetisch wirkt, zur Behandlung bzw. Prophylaxe eines Leidens bzw. einer Erkrankung mit reduzierter cholinerger Funktion ausgewählt aus Alzheimer-Krankheit, Lernschwäche, Konzentrationsstörungen, Angst, Depression, Raucherentwöhnung, Schizophrenie, Tourette-Syndrom und Parkinson-Krankheit.

4. Verwendung einer Kombination nach Anspruch 3 bei der Herstellung eines Medikaments zur Behandlung bzw. Prophylaxe von Alzheimer-Krankheit.

5. Kombination nach Anspruch 1, wobei es sich bei dem Statin um Rosuvastatin oder ein pharmazeutisch annehmbares Salz davon handelt, und
der α7-nAChR-Agonist ausgewählt ist aus:
Spiro[1-azabicyclo[2.2.2]octan-3,5-oxazolidin]-2'-on;
N-(1-Azabicyclo[2.2.2]oct-3-yl)[E-3-(2-thienyl)propenamid], oder
(2'R)-5'-(3-Furanyl)spiro[1-azabicyclo[2.2.2]-octan-3,2'-(3'H)-furo[2,3-b]pyridin] und deren pharmazeutisch annehmbaren Salzen.

6. Pharmazeutische Zusammensetzung, enthaltend eine Kombination nach Anspruch 5 zusammen mit einem pharmazeutisch annehmbaren Verdünnungsmittel oder Träger.

7. Verwendung einer Kombination nach Anspruch 5 bei der Herstellung eines Medikaments, das neuroprotektiv oder analgetisch wirkt, zur Behandlung bzw. Prophylaxe eines Leidens bzw. einer Erkrankung mit reduzierter cholinerger Funktion ausgewählt aus Alzheimer-Krankheit, Lernschwäche, Konzentrationsstörungen, Angst, Depression, Raucherentwöhnung, Schizophrenie, Tourette-Syndrom und Parkinson-Krankheit.

8. Verwendung einer Kombination nach Anspruch 7 bei der Herstellung eines Medikaments zur Behandlung bzw. Prophylaxe von Alzheimer-Krankheit.

## Revendications

1. Combinaison qui comprend:
une statine sélectionnée parmi l'atorvastatine, la cérivastatine, la fluvastatine, la lovastatine, la pravastatine de sodium, la simvastatine et la rosuvastatine, ou un sel pharmaceutiquement acceptable de celles-ci et
un agoniste de α7-nAChR sélectionné parmi
la spiro[1-azabicyclo[2.2.2]octane-3,5'-oxazolidine]-2'-one,
(+)-spiro[1-azabicyclo[2.2.2]octane-3,5'-oxazolidine]-2'-one,
la (-) -spiro[1-azabicyclo[2.2.2]octane-3,5'-oxazolidine] -2'-one,
la spiro[1-azabicyclo[2.2.1]heptane-3,5'-oxazolidine-2'-one],
la 3'-méthylspiro-[1-azabicyclo[2.2.2]octane-3,5'-oxazolidine-2'-one],
la spiro[1-azabicyclo[2.2.2]octane-3,2'-(3'H)-furo[2,3-b]pyridine],
la 5'-bromospiro[1-azabicyclo[2.2.2]octane-3,2'-(3'H)-furo[2,3-b]pyridine],
la 5'-phénylspiro[1-azabicyclo[2.2.2]octane-3,2'-(3'H)-furo[2,3-b]pyridine],
la 5'-nitrospiro[1-azabicyclo[2.2.2]octane-3,2'-(3'H)-furo[2,3-b]pyridine],
la 1'-chlorospiro[1-azabicyclo[2.2.2]octane-3,2'-(3'H)-furo[2,3-b]isoquinoline],
la 5'-(phénylcarboxamido)spiro[1-azabicyclo[2.2.2]octane-3,2'-(3'H)-furo[2,3-b] pyridine],
la 5'-(phénylaminocabonylamino) spiro [1-azabicyclo[2.2.2]octane-3,2'-(3'H)-furo[2,3-b] pyridine],
la 5'-(phénylsulfonylamido) spiro [1-azabicyclo [2.2.2] octane--3, 2' - (3'H) -furo [2, 3-b] pyridine],
la 5-aminospiro [1-azabicyclo [2.2.2] octane-3,2'- (3'H) - furo [2,3-b] pyridine],
la 5'-N-méthylaminospiro[1-azabicyclo[2.2.2]octane-3,2'-(3'H)-furo[2,3-b]pyridine],
la 5'-N,N-diméthylaminospiro[1-azabicyclo [2.2.2] octane-3,2'-(3'H)-furo[2,3-b]pyridine],
la 5'-N,N-diéthylaminospiro[1-azabicyclo[2.2.2]octane-3,2'-(3'H)-furo[2,3-b]pyridine],
la 5'-N-éthylaminospiro[1-azabicyclo[2.2.2]octane-3,2'-(3'H)-furo[2,3-b]pyridine]
la 5' -N-benzylaminospiro [1-aza.bicyclo [2 . 2 . 2] octane-3,2'-(3'H)-furo[2,3-b]pyridine],
la 5' -N-formamidospiro [1-azabicyclo [2 . 2. 2] octane-3, 2' - (3' H) -furo [2, 3-b] pyridine],
la 5'-N-acétamidospiro[1-azabicyclo[2.2.2]octane-3,2'-(3' H) -furo [2, 3-b] pyridine],
la spiro [1-azabicyclo [2.2.2] octane-3,2' - (3'H) -furo [2, 3-b] isoquinoline],
la spiro [1-azabicyclo [2. 2 . 2] octane-3, 2' - (3' H) -furo [2, 3-b] quinoline],
la 5'-éthénylspiro[1-azabicyclo[2.2.2]octane-3,2'-(3'H)-furo[2,3-b]pyridine],
la 5'-(E)-(phényléthényl)spiro[1-azabicyclo[2.2.2]octane-3,2'-(3'H)-furo[2,3-b]pyridine],
la 5'(4-morpholino)spiro[1-azabicyclo[2.2.2]octane-3,2'-(3'H)-furo[2,3-b]pyridine],
la 5'-(1-azétidinyl)spiro[1-azabicyclo[2.2.2]octane-3,2'-(3'H)-furo[2,3-b]pyrldine],
la 5'-(E)-(2-(4-pyridyl)éthényl)spiro[1-azabicyclo[2.2.2]octane-3,2'-(3'H)-furo[2,3-b] pyridine],
la 5'-(E)-(2-2-pyridyl)éthényl)spiro[1-azabicyclo [2.2.2] octane-3, 2' - (3'H) -furo [2, 3-b]pyridine],
la 5'-(2-triméthylsilyléthynyl)spiro[1-azabicyclo[2.2.2]octane-3,2'-(3'H)-furo[2,3-b] pyridine],
la 5'-éthynylspiro[1-azabicyclo[2.2.2]octane-3,2'-(3' H) -furo [2, 3-b] pyridine],
la 5'-(2-furyl)spiro[1-azabicyclo[2.2.2]octane-3,2'-(3'H)-furo[2,3-b]pyridine],
la 5'-(3-pyridyl)spiro[1-azabicyclo[2.2.2]octane-3,2'-(3'H)-furo[2,3-b]pyridine],
la 5'-méthylspiro[1-azabicyclo[2.2.2]octane-3,2'-(3'H)-furo [2, 3-b] pyridine],
le spiro[1-azabicyclo[2.2.2]octane-3,2'-(3'H)-furo[2,3-b]pyridine-5'carbonitrile],
le spixo[1-azabicyclo[2.2.2]octane-3,2'-(3'H)-furo[2,3-b]pyridine-5'carboxamide,
la 5'-N'-(3-chlorophényl)aminocarbonylaminospiro[1-azabicyclo[2.2.2]octane-3,2'-(3'H)-furo[2,3-b] pyridine],
la 5'-N'-(2-nitrophényl)aminocarbonylaminospiro[1-azabicyclo[2.2.2]octane-3,2'-(3'H)-furo[2,3-b] pyridine],
la 4'-chlorospiro[1-azabicyclo[2.2.2]octane-3,2'-(3'H)-furo[2,3-b]pyridine],
la 4'-méthoxyspiro[1-azabicyclo[2.2.2]octane-3,2'-(3'H)-furo[2,3-b]pyridine],
la 4'-phénylthiospiro[1-azabicyclo[2.2.2]octane-3,2'-(3'H)-furo[2,3-b]pyridine],
la 4'-(N-2-aminoéthyl)aminospiro[1-azabicyclo[2.2.2]octane-3,2'-(3'H)-furo[2,3-b]pyridine],
la 4'-phénylaminospiro[1-azabicyclo[2.2.2]octane-3,2'-(3'H)-furo[2,3-b]pyridine],
la 4'-méthylaminospiro[1-azabicyclo[2.2.2]octane-3,2'-(3'H)-furo[2,3-b]pyridine],
la 4'-(4-N-méthylpipérazine-1-yl)spiro[1-azabicyclo[2.2.2]octane-3,2'-(3'H)-furo[2,3-b] pyridine],
la 4'-chlorospiro[1-azabicyclo[2.2.2]octane-3,2'-(3'H)-furo[3,2-c]pyridine],
la spiro[1-azabicyclo[2.2.2]octane-3,2'-(3'H)-furo[3,2-c] pyridine],
le spiro[1-azabicyclo[2.2.2]octane-3,2' (3'H)-furo[2,3-b]pyridine-7'-oxyde,
le spiro[1-azabicyclo[2.2.2]octane-3,2'(3'H)-furo[2,3-b]pyridine-6'-carbonitrile],
la 6'-chlorospiro[1-azabicyclo[2.2.2]octane-3,2'(3'H)-furo[2,3-b]pyridine],
la 6'-fluororospiro[1-azabicyclo[2.2.2]octane-3,2'(3'H)-furo[2,3-b]pyridine],
le *N*-(1-azabicyclo[2.2.2]oct-3-yl)(5-phénylfurane-2-carboxamide),
le *N*-(1-azabicyclo[2.2.2]oct-3-yl)(5-(3-fluorophénylfurane-2-carboxamide),
le *N*-(1-azabicyclo[2.2.2]oct-3-yl)(3-(3-thiényl)benzamide),
le *N*-(1-azabicyclo[2.2.2] oct-3-yl)(3-phénylbenzamide),
le *N*-(1-azabicyclo[2.2.2]oct-3-yl)(3-(3-pyridyl)benzamide),
le *N*-(1-azabicyclo[2.2.2]oct-3-yl)(5-phénylthiophène-2-carboxamide),
le *N*-(1-azabicyclo[2.2.2]oct-3-yl)(3-(3-méthoxyphényl)benzamide),
le *N*-(1-azabicyclo[2.2.2]oct-3-yl)(3-(2-méthoxyphényl)benzamide),
le *N*-(1-azabicyclo[2.2.2]oct-3-yl) (3- (3-N-acétylamino)phényl)benzamide),
le *N*-(1-azabicyclo[2.2.2]oct-3-yl)(3-(3-fluorophényl)benzamide),
le *N*-(1-azabicyclo[2.2.2]oct-3-yl)(3-(3-méthylphényl)benzamide),
le *N*-(1-azabicyclo[2.2.2]oct-3-yl)(3-(2-thiényl)benzamide),
le *N*-(1-azabicyclo[2.2.2]oct-3-yl)(3-(3,5-dichlorophényl)benzamide),
le *N*-(1-azabicyclo[2.2.2]oct-3-yl)(3-(2-naphtyl)benzamide),
le *N*-(1-azabicyclo[2.2.2]oct-3-yl)(3-(4-fluorophényl)benzamide),
le *N*-(1-azabicyclo[2.2.2]oct-3-yl)(5-(3-pyridyl)furane-2-carboxamide),
le *N*-(1-azabicyclo[2.2.2]oct-3-yl)(5-(3-thiényl)furane-2-carboxamide),
le *N*-(1-azabicyclo[2.2.2]oct-3-yl)(5-(2-benzo[b]furanyl)furane-2-carboxamide),
le *N*-(1-azabicyclo[2.2.2]oct-3-yl)(5-(4-pyridyl-furane-2-carboxamide),
le *N*-(1-azabicyclo[2.2.2]oct-3-yl)(5-(2-thiényl)furane-2-carboxamide),
le *N*-(1-azabicyclo[2.2.2]oct-3-yl)(5-(3-méthoxyphényl)furane-2-carboxamide),
le *N*-(1-azabicyclo[2.2.2]oct-3-yl)(5-(2-méthoxyphényl)furane-2-carboxamide),
le *N*-(1-azabicyclo[2.2.2]oct-3-yl)(5-(4-fluorophényl)furane-2-carboxamide),
le *N*-(1-azabicyclo[2.2.2]oct-3-yl)(5-(2-naphtyl)furane-2-carboxamide)
le *N*-(1-azabicyclo[2.2.2]oct-3-yl)(5-(3-méthylphényl)furane-2-carboxamide),
le *N*-(1-azabicyclo[2.2.2]oct-3-yl)(5-(3-furyl)furane-2-carboxamide)
le *N*-(1-azabicyclo[2.2.2]oct-3-yl)(5-(2-furyl)furane-2-carboxamide),
le *N*-(1-azabicyclo[2.2.2]oct-3-yl)(5-(2-pyridyl)furane-2-carboxamide),
le *N*-(1-azabicyclo[2.2.2]oct-3-yl)(5-(4-pyridyl)thiophène-2-carboxamide),
le *N*-(1-azabicycla[2.2.2]oct-3-yl)(5-(3-pyridyl) thiophène-2-carboxamide),
le N- (1-azabicyclo [2 .2.2] oct--3-yl) (5- (2-pyridyl) thiophène-2-carboxamide),
le *N*-(1-azabicyclo[2.2.2]oct-3-yl)(4-(2-pyridyl)thiophène-2-carboxamide),
le *N*-(1-azabicyclo[2.2.2]oct-3-yl) (4-(4-pyridyl)thiophène-2-carboxamide),
le *N*-(1-azabicyclo[2.2.2]oct-3-yl)(4-(3-pyridyl)thiophène-2-carboxamide),
le *N*- (1-azabicyclo [2.2.2] oct-3-yl) (5- (3- (*N-*acétylamino) phényl) furane-2-carboxamide),
le *N*-(1-azabicyclo[2.2.2]oct-3-yl)(5-(3-nitrophényl) furane-2-carboxamide),
le *N*-(1-azabicyclo[2.2.2]oct-3-yl)(5-(3-trifluorométhylphényl) furane-2-carboxamide),
le *N*-(1-azabicyclo[2.2.2]oct-3-yl)(5-(3-chlorophényl)furane-2-carboxamide),
le *N*-(1-azabicyclo[2.2.2]oct-3-yl)(5-(3-(*N-*acétylamino)phényl-thiophène-2-carboxamide),
le *N*-(1-azabicyclo[2.2.2]oct-3-yl)(5-(3-fluorophényl)thiophène-2-carboxamide),
le *N*- (1-azabicyclo [2.2.2] act-3-yl) (5- (3-méthoxyphényl)thiophène-2-carbaxamide),
le *N*-(1-azabicyclo[2.2.2]oct-3-yl)(5-(3-éthoxyphényl) thiophène-2-carboxamide),
le *N*-(1-azabicyclo[2.2.2]oct-3-yl)(5-(3,5-diméthylisoxazole-4-yl)furane-2-carboxamide),
le *N*-(1-azabicyclo[2.2.2]oct-3-yl)(5-(3,5-diméthylisoxazole-4-yl)thiophène-2-carboxamide),
le *N*-(1-azabicyclo[2.2.2]oct-3-yl)(5-(3-aminophényl)thiophène-2-carbaxamide),
le *N*-(1-azabicyclo[2.2.2]oct-3-yl)(5-(3-pyridyl-thiophène-3-carbaxamide),
le *N*-(1-azabicyclo[2.2.2]oct-3-yl)(5-(4-chlorophényl) furane-2-carboxamide),
le *N*-(1-azabicyclo[2.2.2]oct-3-yl)(5-(3-pyridyl)thioazole-3-carboxamide),
le *N*-(1-azabicyclo[2.2.2]oct-3-yl)(5-(4-pyridyl)thioazole-3-carboxamide),
le *N*- (1-azabicyclo[2.2.2]oct-3-yl) (5- (3- *(N,N-*diméthylamino)phényl)thiophène-2-carboxamide),
le *N*-(1-azabicyclo[2.2.2]oct-3-yl)(5-(8-quinolinyl)thiophène-2-carboxamide),
le *N*-(1-azabicyclo[2.2.2]oct-3-yl)(5-phénylthiophène-3-carboxamide),
le *N*-(1-azabicyclo [2 .2 .2] oct-3-yl) (4-phénylthiophène-2-carboxamide),
le *N*- (1-azabicyclo [2.2.2] oct-3-yl) (5- (3-cyanophényl)thiophène-2-carboxamide),
le *N*-(1-azabicyclo[2.2.2]oct-3-yl)(5-(3-(*N-*méthylamino)phényl)thiophène-2-carboxamide),
le *N*-(1-azabicyclo[2.2.2]oct-3-yl)(5-(3-hydroxyphényl)thiophène-2-carboxamide,
le *N*-(1-azabicyclo[2.2.2]oct-3-yl)(5-(3-pyridylamino)thiophène-2-carboxamide,
le *N*-(1-azabicyclo[2.2.2]oct-3-yl)(5-(3-chlorophényl)thiophène-2-carboxamide,
le *N-*(1-azabicyclo[2.2.2]oct-3-yl)(5-(3-(4-morpholinyl)phényl)thiophène-2-carbosamide),
le *N*-(1-azabicyclo[2.2.2]oct-3-yl)(5-(3-(aminométhyl)phényl)thiophène-2-carboxamide),
le *N*-(1-azabicyclo[2.2.2]oct-3-yl)(5-phénoxythiophène-2-carboxamide),
le *N*-(1-azabicyclo[2.2.2]oct-3-yl)(5-(3-aminophényl)furane-2-carboxamide),
le *N-* (1-azabicyclo[2.2.2]oct-3-yl) (5- (3- *(N, N-*dimëthylamino-phënyl)furane-2-carboxamide),
le *N*-(1-azabicyclo[2.2.2]oct-3-yl)(5-(3-formylphényl)thiophène-2-carboxamide),
le *N*-(1-azabicyclo[2.2.2]oct-3-yl)(5-(3-(hydroxyméthyl)phényl)thiophène-2-carboxamide),
le (*R*)-*N*-(1-azabicyclo[2.2.2]oct-3-yl)(5-phénylfurane-2-carboxamide)
le (*R*)-*N*-(1-azabicyclo[2.2.2]oct-3-yl)(5-(3-fluorophényl)furane-2-carboxamide),
le (*R*)-*N*-(1-azabicyclo[2.2.2]oct-3-yl)(3-(3-thiényl)benzamide),
le (*R*)-*N*-(1-azabicyclo[2.2.2]oct-3-yl)(3-phénylbenzamide),
le (*R*)-*N*-(1-azabicyclo[2.2.2]oct-3-yl)(3-(3-pyridyl)benzamide),
le (*R*)-*N*-(1-azabicyclo[2.2.2]oct-3-yl)(5-phénylthiophéne-2-carboxamide),
le (*R*) -*N*- (1-azabicyclo[2.2.2]act-3-yl) (3- (3-méthoxyphényl)benzamide),
le (*R*)-*N*-(1-azabicyclo[2.2.2]oct-3-yl)(3-(2-méthoxyphéyl)benzamide
le (*R*)-*N*-(1-azabicyclo[2.2.2]oct-3-yl)(3-(3-(*N-*acétylamino)phényl)benzamide),
le (*R*)-*N*-(1-azabicyclo[2.2.2]oct-3-yl)(3-(3-fluorophényl)benzamide),
le (*R*)-*N*-(1-azabicyclo[2.2.2]oct-3-yl)(3-(3-méthylphényl)benzamide),
le (*R*)-*N*-(1-azabicyclo[2.2.2]oct-3-yl) (3- (2-thiényl)benzamide),
le (*R*)-*N*-(1-azabicyclo[2.2.2]oct-3-yl)(3-(3,5-dichlorophényl)benzamide),
le (*R*)-*N*-(1-azabicyclo[2.2.2]oct-3-yl)(3-(2-naphtyl)benzamide),
le (*R*)-*N*-(1-azabicyclo[2.2.2]oct-3-yl)(3-(4-fluorophényl)benzamide),
le (*R*)-*N*-(1-azabicyclo[2.2.2]oct-3-yl)(5-(3-pyridyl)furane-2-carboxamide),
le (*R*) -*N*- (1-azabicyclo [2.2.2] oct-3-yl) (5- (3-thiényl)furane-2-carboxamide),
le (*R*)-*N*-(1-azabicyclo[2.2.2]oct-3-yl)(5-(2-benzo[b]furanyl)furane-2-carboxamide),
le (*R*)-*N*-(1-azabicyclo[2.2.2]oct-3-yl)(5-(4-pydidyl)furane-2-carboxamide),
le (*R*)-*N*-(1-azabicyclo[2.2.2]oct-3-yl(5-(2-thiényl)furane-2-carboxamide,
le (*R*)-*N*-(1-azabicyclo[2.2.2]oct-3-yl)(5-(3-méthoxyphényl)furane-2-carboxamide),
le (*R*)-*N*-(1-azabicyclo[2.2.2]oct-3-yl)(5-(2-méthoxyphényl)furane-2-carboxamide),
le (*R*)-*N*-(1-azabicyclo[2.2.2]oct-3-yl)(5-(4-fluorophényl)furane-2-carboxamide),
le (*R*)-*N*-(1-azabicyclo[2.2.2]oct-3-yl)(5-(2-naphtyl)furane-2-carboxamide),
le (*R*)-*N*-(1-azabicyclo[2.2.2]oct-3-yl)(5-(3-méthylphényl)furane-2-carboxamide),
le (*R*)-*N*-(1-azabicyclo[2.2.2]oct-3-yl)(5-(3-furyl)furane-2-carboxamide),
le (*R*)-*N*-(1-azabicyclo[2.2.2]oct-3-yl)(5-(2-furyl)furane-2-carboxamide),
le (*R*)-*N*-(1-azabicyclo[2.2.2]oct-3-yl)(5-(2-pyridyl)furane-2-carboxamide),
le (*R*)-*N*-(1-azabicyclo[2.2.2]oct-3-yl)(5-(4-pyridyl)thiophène-2-carboxamide),
le (*R*)-*N*-(1-azabicyclo[2.2.2]oct-3-yl)(5-(3-pyridyl)thiophène-2-carboxamide),
le (*R*)-*N*-(1-azabicyclo[2.2.2]oct-3-yl)(5-(2-pyridyl)thiophène-2-carboxamide),
le (*R*)-*N*-(1-azabicyclo[2.2.2]oct-3-yl)(4-(2-pyridyl)thiophène-2-carboxamide),
le (*R*)-*N*-(1-azabicyclo[2.2.2]oct-3-yl)(4-(4-pyridyl)thiophène-2-carboxamide),
le (*R*)-*N*-(1-azabicyclo[2.2.2]oct-3-yl)(4-(3-pyridyl)thiophène-2-carboxamide),
le (*R*)-*N*-(1-azabicyclo[2.2.2]oct-3-yl)(5-(3-(*N-*acétylamino)phényl)furane-2-carboxamide),
le (*R*)-*N*-(1-azabicyclo[2.2.2]oct-3-yl) (5-(3-nitrophényl)furane-2-carboxamide),
le (*R*)*-N-*(1-azabicyclo[2.2.2]oct-3-yl)(5-(3-trifluorométhylphényl)furane-2-carboxamide),
le (*R*)-*N*-(1-azabicyclo[2.2.2]oct-3-yl)(5-(3-chlorophényl)furane-2-carboxamide),
le (*R*)-*N*-(1-azabicyclo[2.2.2]oct-3-yl)(5-(3-*N-*acétylamino)phényl)thiophène-2-carboxamide),
le (*R*)-*N*-(1-azabicyclo[2.2.2]oct-3-yl)(5-(3-fluorophényl)thiophène-2-carboxamide),
le (*R*)-*N*-(1-azabicyclo[2.2.2]oct-3-yl)(5-(3-méthoxyphényl)thiophène-2-carboxamide),
le (*R*)-*N*-(1-azabicyclo[2.2.2]oct-3-yl) (5-3-éthoxyphényl)thiophène-2-carboxamide),
le (*R*)-*N*-(1-azabicyclo[2.2.2]oct-3-yl)(5-(3,5-diméthylisoxazole-4-yl)furane-2-carboxamide),
le (*R*)-*N*-(1-azabicyclo[2.2.2]oct-3-yl)(5-(3,5-diméthylisoxazole-4-yl)thiophène-2-carboxamide) ,
le (*R*)-*N*-(1-azabicyclo[2.2.2]oct-3-yl)(5-(3-aminophényl)thiophène-2-carboxamide),
le (*R*)-*N*-(1-azabicyclo[2.2.2]oct-3-yl) (5-(3-pyridyl)thiophène-3-carboxamide),
le (*R*)-*N*-(1-azabicyclo[2.2.2]oct-3-yl)(5-(4-chlorophényl)furane-2-carboxamide),
le (*R*)-*N*-(1-azabicyclo[2.2.2]oct-3-yl)(5-(3-pyridyl)thiazole-3-carboxamide),
le (*R*)-*N*-(1-azabicyclo[2.2.2]oct-3-yl)(5-(4-pyridyl)thiazole-3-carboxamide),
le (*R*) -*N*- (1-azabicyclo[2.2.2]oct-3-yl) (5- (3- (*N*,*N-*diméthylamino)phényl)thiophène-2-carboxamide),
le (*R*)-*N*-(1-azabicyclo[2.2.2]oct-3-yl)(5-(8-quinolinyl)thiophène-2-carboxamide),
le (*S*)-*N*-(1-azabicyclo[2.2.2]oct-3-yl)(5-(3-pyridyl)thiophène-2-carboxamide),
le (*S*)*-N-*(1-azabicyclo[2.2.2]oct-3-yl)(5-(4-pyridyl)thiophène-2-carboxamide),
le (*S*)-*N*-(1-azabicyclo[2.2.2]oct-3-yl) (5-2-pyridyl)thiophéne-2-carboxamide),
le (*S*)-*N*-(1-azabicyclo[2.2.2]oct-3-yl)(5-phénylthiophène-2-carboxamide),
le (*R*)-*N*-(1-azabicyclo[2.2.2]oct-3-yl)(5-phénylthiophène-3-carboxamide),
le (*R*)-*N*-(1-azabicyclo[2.2.2]oct-3-yl)(4-phénylthiophène-2-carboxamide),
le (*R*)-*N*- (1-azabicyclo [2.2.2] oct-3-yl) (5- (3-cyanophényl) thiophène-2-carboxamide),
le (*R*)-*N*-(1-azabicyclo[2.2.2]oct-3-yl) (5-3 (*N-*méthylamino)phényl)thiophène-2-carboxamide),
le (*R*)-*N*-(1-azabicyclo[2.2.2]oct-3-yl)(5-(3-hydroxyphényl)thiophène-2-carboxamide),
le (*R*)-*N*-(1-azabicyclo[2.2.2]oct-3-yl)(5-(3-pyridylamino)thiophène-2-carboxamide),
le (*R*)-*N*-(1-azabicyclo[2.2.2]oct-3-yl)(5-(3-chlorophényl) thiophène-2-carboxamide),
le (*R*) -*N*- (1-azabicyclo [2.2.2] oct-3-yl) (5- (3- (4-morpholinyl)phényl)thiophène-2-carboxamide),
le (*R*)-*N*-(1-azabicyclo[2.2.2]oct-3-yl)(5-(3-aminométhyl)phényl)thiophène-2-carboxamide),
le (*R*)-*N*-(1-azabicyclo[2.2.2]oct-3-yl)(5-phénoxythiophène-2-carboxamide),
le (*R*)-*N*-(1-azabicyclo[2.2.2]oct-3-yl)(5-(3-aminophényl)furane-2-carboxamide),
le (*R*)-*N*-(1-azabicyclo[2.2.2]oct-3-yl) (5-(3-*N*,*N-*diméthylamino)phényl)furane-2-carboxamide,
le (*R*)-*N*-(1-azabicyclo[2.2.2]oct-3-yl)(5-(3-formylphényl)thiophène-2-carboxamide), ou
le (*R*)-*N*-(1-azabicyclo[2.2.2]oct-3-yl)(5-(3-(hyroxyméthyl)phényl)thiophène-2-carboxamide), ou un sel pharmaceutiquement acceptable de ceux-ci.

2. Composition pharmaceutique qui contient une combinaison selon la revendication 1 en même temps qu'un diluant ou porteur pharmaceutiquement acceptables.

3. Utilisation d'une combinaison selon la revendication 1 pour la préparation d'un médicament qui assure une neuroprotection ou une analgésie pour le traitement ou la prophylaxie d'un état ou d'un trouble qui impliquent une réduction de la fonction cholinergique, sélectionnés parmi la maladie d'Alzheimer, les troubles cognitifs ou de l'attention, l'anxiété, la dépression, le sevrage du tabac, la schizophrénie, le syndrome de Tourette et la maladie de Parkinson.

4. Utilisation d'une combinaison selon la revendication 3 dans la préparation d'un médicament destiné au traitement ou à la prophylaxie de la maladie d'Alzheimer.

5. Combinaison selon la revendication 1, dans laquelle ladite statine est la rosuvastatine ou un sel pharmaceutiquement acceptable de celle-ci, et dans laquelle ledit agoniste de la α7-nAChR est sélectionné parmi:
la spiro[1-azabicyclo[2.2.2]octane-3,5'-oxazolidine]-2'-one,
le *N*-(1-azabicyclo[2.2.2]oct-3-yl)[*E*-3-(2-thiënyl)propénamide] ou
la (2'R)-5'-(3-furanyl)spiro[1-aaabicyclo[2.2.2]octane-3,2'-(3'H)-furo[2,3-b]pyridine], ou un sel pharmaceutiquement acceptable de ceux-ci.

6. Combinaison pharmaceutique qui comprend une combinaison selon la revendication 5 ainsi q'un diluant ou porteur pharmaceutiquement acceptables.

7. Utilisation d'une combinaison selon la revendication 5 dans la préparation d'un médicament assurant une neuroprotection ou une analgésie pour le traitement ou la prophylaxie d'une affection ou d'un trouble entraînant une fonction cholinergique réduite, comme la maladie d'Alzheimer, les troubles cognitifs ou de l'attention, l'anxiété, la dépression, l'arrêt de fumer, la schizophrénie, le syndrome de Tourette, et la maladie de Parkinson.

8. Utilisation d'une combinaison selon la revendication 7 dans la préparation d'un médicament destiné au traitement ou à la prophylaxie de la maladie d'Alzheimer.
